# EUROPEAN PATENT SPECIFICATION

(11) **EP 2 455 760 B1**
(45) Date of publication and mention of the grant of the patent: **13.04.2016**
(21) Application number: 10799578.9
(22) Date of filing: 01.07.2010
(51) Int. Cl.: B01L 3/00, G01N 21/59, G01N 33/92, G01N 35/00

(54) **ANALYSIS REAGENT AND ANALYSIS DEVICE HAVING THE ANALYSIS REAGENT CARRIED THEREIN**
ANALYSEREAGENZ UND ANALYSEVORRICHTUNG MIT DEM ANALYSEREAGENZ DARIN
RÉACTIF D'ANALYSE ET DISPOSITIF D'ANALYSE COMPORTANT LE RÉACTIF D'ANALYSE PORTÉ DANS CELUI-CI

(30) Priority: 15.07.2009 JP 2009166211; 28.08.2009 JP 2009197508
(43) Date of publication of application: 23.05.2012
(73) Proprietor: Panasonic Healthcare Holdings Co., Ltd., Tokyo 105-8433 (JP)
(72) Inventor: MARUYAMA, Yuki, Toon-shi, Ehime 791-0395 (JP); ISHIBASHI, Kenji, Toon-shi, Ehime 791-0395 (JP); SAIKI, Hiroshi, Toon-shi, Ehime 791-0395 (JP); WATANABE, Kenji, Toon-shi, Ehime 791-0395 (JP); TAGASHIRA, Kouzou, Toon-shi, Ehime 791-0395 (JP)
(74) Representative: Grünecker Patent- und Rechtsanwälte PartG mbB
(86) International application number: PCT/JP2010/004325
(87) International publication number: WO 2011/007514

(56) References cited:
- EP-A1- 0 064 665
- WO-A1-00/17388
- WO-A1-03/016325
- WO-A1-03/056163
- WO-A1-2009/037784
- JP-A- 4 009 353
- JP-A- 2006 111 616
- JP-A- 2007 315 879
- JP-T- 2007 523 325
- BURSTEIN M ET AL: "RAPID METHOD FOR THE ISOLATION OF LIPOPROTEINS FROM HUMAN SERUM BY PRECIPITATION WITH POLYANIONS", JOURNAL OF LIPID RESEARCH, AMERICAN SOCIETY FOR BIOCHEMISTRY AND MOLECULAR BIOLOGY, INC, US, vol. 11, 1 January 1970 (1970-01-01), pages 583-595, XP000917101, ISSN: 0022-2275

## Description

### Technical Field

The present invention relates to a reagent for accurately analyzing high-density lipoprotein cholesterol of a biological sample and an analysis device used for analyzing a liquid sample.

### Background Art

Conventionally, large-size automatic analyzers have been practically used which can react a biological sample such as blood with an analysis reagent with a single unit and determine quantities of various components in the biological sample. Such analyzers have been indispensable in the field of medical treatment.

Unfortunately, such analyzers have not been introduced in all hospitals. Particularly, a number of small medical facilities such as clinics outsource sample analysis for various reasons, for example, the operation cost. In outsourcing of analysis, it takes a long time to obtain an analysis result, so that a patient is inconveniently forced to revisit a medical facility to receive proper medical treatment based on a test result. Moreover, quick response is difficult in an emergency case.

Against this backdrop, analyzers with higher accuracy and higher flexibility in operation have been required in actual clinical use. For example, lower cost, a reduction in the quantity of sample liquid, smaller-size analyzers, and a short measurement time have been demanded. Such a prompt and simple diagnosis system is called point-of-care testing (hereinafter, will be called POCT) by which a test result can be quickly obtained near a subject when a test is necessary. POCT is defined as a test aimed at improving the quality of medical treatment and the quality of life of patients.

Ideally, in order to obtain an analyzer that can improve flexibility in operation and the quality of medical service as defined in POCT, the following condition is satisfied: the concentrations of multiple components are accurately measured in a short time from a small quantity of specimen, for example, blood collected from a fingertip with only a small burden on a patient. The quantity of specimen such as blood obtained from a fingertip without stress is, however, not more than ten microliters. It is technically difficult to satisfy the foregoing condition, particularly, accurately analyze multiple components from such a small quantity of specimen. Particularly, for analysis items requiring pretreatment, it is difficult to conduct pretreatment on a small quantity of specimen in a short time with high repeatability. Thus, only a small number of products have sufficient accuracy of measurement under the current circumstances.

In Patent Literature 1, a reagent for pretreatment is carried in a porous body. A specimen is pretreated (cytapheresis, precipitation, and separation treatment) by passing the specimen through the porous body, and then high-density lipoprotein cholesterol (hereinafter, will be called HDL cholesterol) is measured.

In the case where HDL (High Density Lipoprotein) cholesterol is measured in blood, components unnecessary for analysis in blood (non-HDL components) are coagulated and precipitated to separately measure components necessary for analysis (HDL components).

FIG. 28 shows an analysis device using a membrane filter described in Patent Literature 2 and so on. In the analysis device, a separation layer 303, a first carrier 304, and a second carrier 305 are stacked on a test film 306 that reacts with an HDL component to develop a color.

When the blood of a liquid sample is attached to the separation layer 303, blood cell components in the blood are captured by the separation layer 303 and then plasma components penetrate the first carrier 304. The first carrier 304 carries a reagent for coagulating non-HDL components. Non-HDL components are coagulated by passing the plasma components through the first carrier 304. out of the HDL components having passed through the first carrier 304 and the coagulated non-HDL components, only the coagulated non-HDL components are trapped by the second carrier 305 while only components not containing the non-HDL components pass through the second carrier 305 and penetrate the test film 306. A coloring state of the test film 306 that has developed a color in reaction to the HDL components is measured through a film 307 and a quantitative measurement is conducted on the HDL components. Reference number 302 denotes a casing.

Patent Literature 3 discloses a dry phase test strip for the determination of HDL cholesterol concentration in a whole blood sample, the dry phase test strip having a two layer configuration comprising a first separation layer (38) and a second separation layer (40), which both consist of impregnated glass fiber matrices. The first separation layer 38 has the function of separating most of the blood cells, which may be facilitated by impregnating the layer 38 with a salt (with NaCl, KCl, MgCl₂, CsCl₂, Li₂SO₄, CaCl₂, Rb₂SO₄, CsSO₄ and amino acids_Gly Ala Asp, Glu and glycinamide asparagine being listed as examples) and a sugar. The second separation layer 40 is used for single layer precipitation and separation of non-HDLs, which is achieved by addition of a polyanion and a bivalent cation.

### Citation List

### Patent Literature

Patent Literature 1: Japanese Patent Publication No. 7-66001
Patent Literature 2: US 6,171,849 B1
Patent Literature 3: WO 03/056163 A1

### Summary of Intention

### Technical Problem

Cholesterol in blood circulates a body as the components of lipoprotein that is a composite of lipid and protein. Lipoprotein is broadly categorized by a difference in specific gravity into chylomicron, very low density lipoprotein, low density lipoprotein, and high density lipoprotein (hereinafter, will be called HDL) in ascending order of specific gravity. Cholesterol contained in HDL is HDL cholesterol. It is known that HDL cholesterol is generally called "good" cholesterol, a negative factor of arteriosclerosis. Hence, HDL cholesterol is analyzed mainly for evaluating the risk of arteriosclerosis and screening lipidosis.

A method of measuring HDL cholesterol can be broadly categorized into two methods.

In a first analysts method, lipoprotein other than HDL is precipitated and removed, and then HDL cholesterol contained in residual HDL is analyzed. Unfortunately, this method requires complicated manual pretreatment. As in Patent Literature 1, a device of so-called dry chemistry is available in which a pretreatment reagent is carried in a small porous body and then a small quantity of specimen is passed through the porous body, so that a precipitation is automatically generated and removed. Since the porous body and a capillary force are used in pretreatment reaction, a precipitation is likely to be unevenly generated and removed because of variations in physical shape, for example, the pore size of the porous body, the concentration gradient of the pretreatment reagent between the end of a specimen previously introduced into the porous body and a specimen introduced thereafter, and variations in the physical characteristics of the specimens. Moreover, the pretreatment reagent is typically composed of polyanion and bivalent cation and is disadvantageous in deliquescence and solubility in a dry state.

In a second analysis method, the reaction of lipoprotein cholesterol other than HDL cholesterol is blocked by using a specific polymeric material or surface-active agent, so that a pretreatment process including the generation and removal of a precipitation is omitted. This technique is called homogeneous method that is currently used as a main analysis method of HDL cholesterol. This method has been developed for large-size automatic analyzers and thus it is difficult to introduce the method in all medical facilities because the method requires large analyzers and high operation cost. Moreover, the method is designed on the assumption that the reagent is a liquid. Thus, the use of the method requires a large number of mechanical mechanisms precluding the size reduction of an analyzer, which is disadvantageous to operations in POCT. Currently, operations defined in POCT cannot be performed.

In order to improve the quality of medical service and the quality of life of patients as defined in POCT, we concluded that a system is necessary that can analyze a blood specimen of approximately ten microliters or less from a fingertip so as to reduce the burden of a patient and can accurately measure target components in blood in a short time, e.g., several minutes. A dry-chemistry measurement system is selected as a method for achieving the system. An object of the present invention is to provide a pretreatment reagent by which an HDL cholesterol concentration in blood can be accurately measured in a short time with extremely high solubility and stability according to the method.

Moreover, in Patent Literature 2, the membrane includes the two layers that are the first carrier 304 carrying the reagent and the second carrier 305 having the function of separating non-HDL components. Such a complicated configuration may lead to variations in measurement results.

Furthermore, when blood is attached to the separation layer 303 in contact with the reagent for coagulating non-HDL, the solubility of the reagent may have a distribution. Moreover, it may take a long time to generate non-HDL coagulated components and correct values may not be obtained because of insufficient treatment.

A particular problem of the membrane filter is that some of HDL components required for measurement are likely to be trapped by the second carrier 305, leading to a large loss of the liquid sample. Hence, an extremely large quantity of liquid sample needs to be prepared, causing a large burden on a subject.

An object of the present invention is to provide an analysis device and an analysis method by which correct values can be obtained even in a short time with small variations in measurement results and a small loss of a liquid sample.

### Solution to Problem

An analysis reagent of the present invention is an analysis reagent that coagulates lipoprotein other than high-density lipoprotein in an analysis of high-density lipoprotein cholesterol contained in a biological sample, wherein the reagent including a combination of a polyanionic compound and a bivalent cationic compound contains one substance selected from the group consisting of succinic acid, gluconic acid, alanine, glycine, valine, histidine, maltitol, and mannitol or a salt thereof, wherein the polyanionic compound is at least one selected from the group consisting of phosphotungstic acid, phosphotungstate, phosphomolybdic acid, and molybdophosphate; and wherein the bivalent cationic compound is at least one selected from the group consisting of a magnesium ion compound, magnesium ion compound salt, a calcium ion compound, and calcium ion compound salt.

An analysis device of the present invention is an analysis device having a microchannel structure for transferring a sample liquid to a measuring cell by a centrifugal force, the analysis device being used for reading that accesses a reaction liquid in the measuring cell, wherein an analysis reagent including a combination of a polyanionic compound and a bivalent cationic compound in a solid state is carried in the passage of the microchannel structure before reaching the measuring cell, the reagent containing one substance selected from the group consisting of succinic acid, gluconic acid, alanine, glycine, valine, histidine, maltitol, and mannitol or a salt thereof.

### Advantageous Effects of Invention

An analysis reagent of the present invention contains at least one compound selected from succinic acid, alanine, glycine, valine, histidine, maltitol, and mannitol, thereby achieving a pretreatment reagent with higher solubility, short-time pretreatment, and uniform treatment less affected by a concentration gradient and physical characteristics varied among specimens. Thus, HDL cholesterol can be accurately measured in a short time.

Specifically, the analysis reagent of the present invention is based on a known technique using polyanion and bivalent cation. The analysis reagent is, however, disadvantageous in deliquescence and solubility in a dry state and thus cannot solve the problem. In order to solve the problem, deliquescence is reduced and solubility is improved in a dry state.

In order to reduce the deliquescence of the pretreatment reagent and improve solubility, it is important to select the salts of reagent components and additives. This is because deliquescence and solubility largely vary depending on the type of salts and additives. Regarding the type of salts of reagent components, for example, sulfate tends to be less deliquescent than hydrochloric, though the same does not hold true for all compounds. The additives improve solubility by changing the crystalline state of a dried reagent mixture from, for example, a monocrystalline state in which large crystals are precipitated disadvantageously to solubility to a polycrystalline state in which fine crystals are precipitated advantageously to solubility, an amorphous state, or a non-crystalline state. Furthermore, the additives reduce deliquescence by a coating effect that captures highly deliquescent reagent components into the crystalline structures of the additives. Only for the function of precipitating non-HDL, a polyanionic compound is selected from phosphotungstic acid, phosphomolybdic acid, and the salts thereof. Furthermore, phosphotungstate is preferable. Only for the function of precipitating non-HDL, a bivalent cationic compound is selected from calcium or magnesium ions in order to satisfy the conditions. Moreover, as a compound, calcium sulfate and magnesium sulfate are desirably selected and combined. Non-HDL can be precipitated by combining the polyanionic compound and the bivalent cationic compound. As has been discussed, additives need to be added to satisfy solubility in a solid state and the condition of reducing deliquescence. Desirable additives are saccharides, amino acids, dicarboxylic acids in a solid state at room temperature, or the salts thereof. Moreover, desirable saccharides are mannitol and maltitol. Desirable amino acids are alanine, glycine, and histidine. Desirable dicarboxylic acids are succinic acids or the salts thereof. Disodium succinate is the most suitable for the combination of polyanion and a bivalent cationic compound and the analysis device of the present invention.

The pretreatment reagent composed of the combination achieves low deliquescence in a dry state and extremely high solubility in contact with a biological sample. An analysis system using the pretreatment reagent makes it possible to measure HDL cholesterol according to the definition of POCT.

In the analysis device of the present invention, a reserving cavity, an operation cavity containing the reagent for analyzing HDL cholesterol, a separating cavity, measuring passages, measuring cells, and capillary areas containing an enzyme reagent and a mediator are formed by a microchannel structure. A centrifugal force is controlled so as to perform transportation, mixing/agitation with the reagent, and separation with a small loss of liquid sample. Furthermore, a correct value can be obtained even in a short time.

### Brief Description of Drawings

[FIG. 1] FIG. 1 is a perspective view showing an analysis device with an opened and closed protective cap according to a first embodiment of the present invention.
[FIG. 2] FIG. 2 is an exploded perspective view showing the analysis device according to the first embodiment.
[FIG. 3] FIG. 3 is an enlarged perspective view showing a base substrate according to the first embodiment.
[FIG. 4] FIG. 4 shows a plan view, an A-A sectional view, a side view, a rear view, and a front view of a diluent container according to the first embodiment.
[FIG. 5] FIG. 5 shows a plan view, a side view, a B-B sectional view, and a front view of the protective cap according to the first embodiment.
[FIG. 6] FIG. 6 shows sectional views of the closed diluent container, the opened protective cap, and a discharged diluent according to the first embodiment.
[FIG. 7] FIG. 7 is a sectional view showing a step of setting the analysis device in a shipment state according to the first embodiment.
[FIG. 8] FIG. 8 is a perspective view showing an analyzing apparatus with an opened door according to the first embodiment.
[FIG. 9] FIG. 9 is a sectional view showing the analyzing apparatus according to the first embodiment.
[FIG. 10] FIG. 10 is a structural diagram of the analyzing apparatus according to the first embodiment.
[FIG. 11] FIG. 11 shows an enlarged perspective view of a portion around the inlet of the analysis device, a perspective view showing that the protective cap is opened and a sample liquid is collected from a fingertip, and an enlarged perspective view of the microchannel structure of the analysis device that is viewed from the turntable through a cover substrate.
[FIG. 12] FIG. 12 is a state diagram showing a state before the analysis device containing the dropped sample liquid is set on the turntable according to the first embodiment.
[FIG. 13] FIG. 13 shows a state diagram in which the analysis device retaining the sample liquid in a capillary cavity is set on the turntable with a broken aluminum seal of a diluent solution, and a state diagram showing the analysis device is separated from the turntable according to the first embodiment.
[FIG. 14] FIG. 14 is an enlarged sectional view for explaining the discharge of a liquid from the diluent container according to the first embodiment.
[FIG. 15] FIG. 15 shows a state diagram in which the sample liquid flows into a measuring passage from a separating cavity and a fixed quantity of the sample liquid is retained in the measuring passage in step 3, and a state diagram in which the sample liquid flows into a mixing cavity from the measuring passage in step 4 according to the first embodiment.
[FIG. 16] FIG. 16 shows a state diagram of the analysis device oscillated in step 6 of the first embodiment, and a state diagram in which the turntable is rotationally driven in a clockwise direction to cause the sample liquid to flow into a measuring cell and a reserving cavity.
[FIG. 17] FIG. 17 shows a state diagram of the analysis device oscillated in step 8 of the first embodiment, and a state diagram in which the turntable is rotationally driven in the clockwise direction in step 9 to cause diluted plasma having reacted with the reagent of an operation cavity to flow into the separating cavity, and aggregates generated in the operation cavity are centrifugally separated by keeping a high-speed rotation.
[FIG. 18] FIG. 18 shows a state diagram in which the turntable is stopped, the diluted plasma flows into the measuring passage, and a fixed quantity of the diluted plasma is retained in the measuring passage in step 10 of the first embodiment, and a state diagram in which the diluted plasma retained in the measuring passage flows into the measuring cell in step 11.
[FIG. 19] FIG. 19 shows a state diagram in which a reaction of the diluted plasma in the measuring cell and reagents is started in step 12 of the first embodiment, and a state diagram of the agitation of the reagents and the diluted plasma in step 13.
[FIG. 20] FIG. 20 shows an enlarged perspective view in which the diluent from the diluent container flows into the reserving cavity through a discharging passage in step 2 of the first embodiment, and an enlarged perspective view in which the diluted plasma is transferred from the mixing cavity to the subsequent process through a capillary passage.
[FIG. 21] FIG. 21 shows a plan view of the analysis device when the turntable is stopped around 180° and a plan view of the analysis device when the turntable is stopped around 60° and 300°.
[FIG. 22] FIG. 22 is a sectional view of the analysis device taken along line F-F of FIG. 16 according to the first embodiment.
[FIG. 23] FIG. 23 shows an enlarged plan view of a state of the reagents contained in capillary areas of the analysis device and a G-G sectional view according to the first embodiment.
[FIG. 24] FIG. 24 shows an enlarged plan view of a state of the reagents in the operation cavity of the analysis device and an H-H sectional view according to the first embodiment.
[FIG. 25] FIG. 25 is an explanatory drawing showing the experimental results of a reference reagent and reagents prepared by adding various additives to the reference reagent according to a second embodiment of the present invention.
[FIG. 26] FIG. 26 is an analysis flowchart of HDL cholesterol in the analysis device containing the reagents of the present invention.
[FIG. 27] FIG. 27 is an explanatory drawing showing the linearity of the measured values of HDL cholesterol in the analysis device.

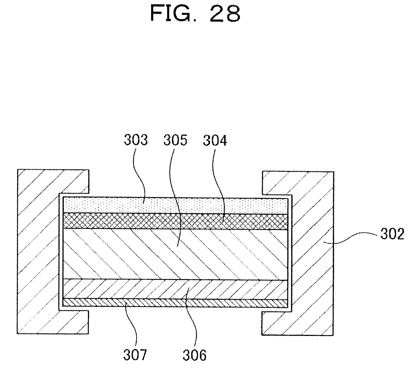

[FIG. 28] FIG. 28 is a structural diagram of Patent Literature 2.

### Description of Embodiments

### (First Embodiment)

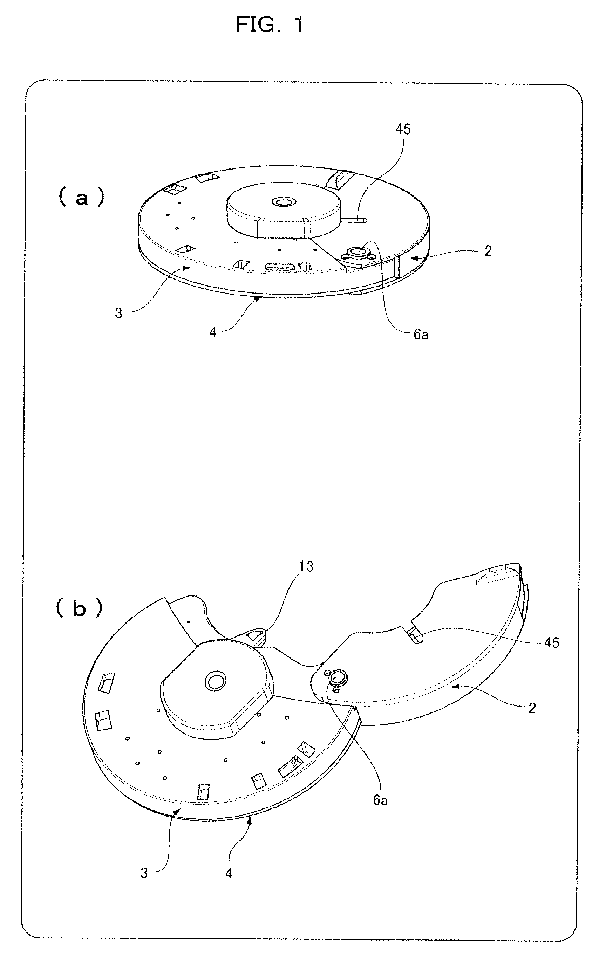

FIGS. 1 to 7 illustrate an analysis device of the present invention.

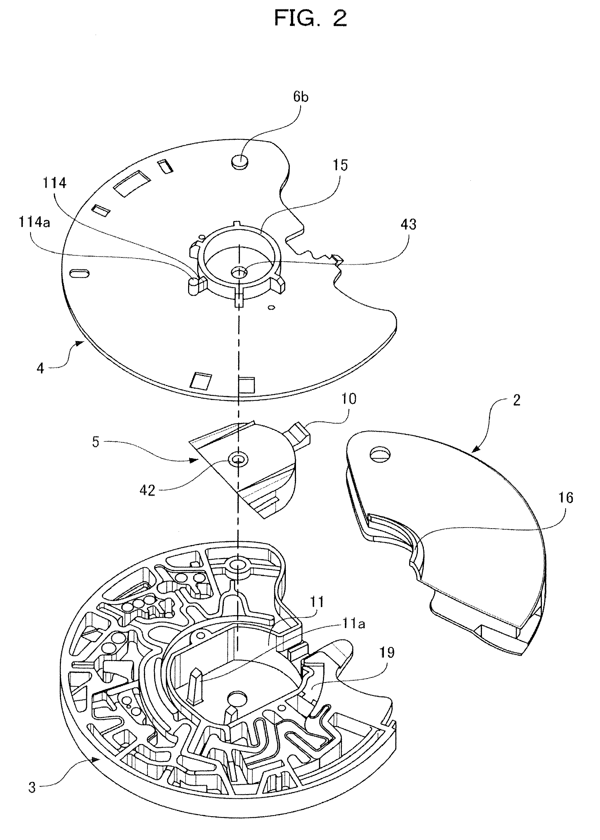

FIGS. 1(a) and 1(b) illustrate an analysis device 1 with an opened and closed protective cap 2. FIG. 2 is an exploded view of the analysis device 1 with the underside of FIG. 1(a) placed face up.

The analysis device 1 includes four components that are a base substrate 3 having a microchannel structure formed on one surface of the base substrate 3, the microchannel structure having a minutely uneven surface, a cover substrate 4 covering the surface of the base substrate 3, a diluent container 5 for retaining a diluent, and the protective cap 2 for preventing splashes of a sample liquid.

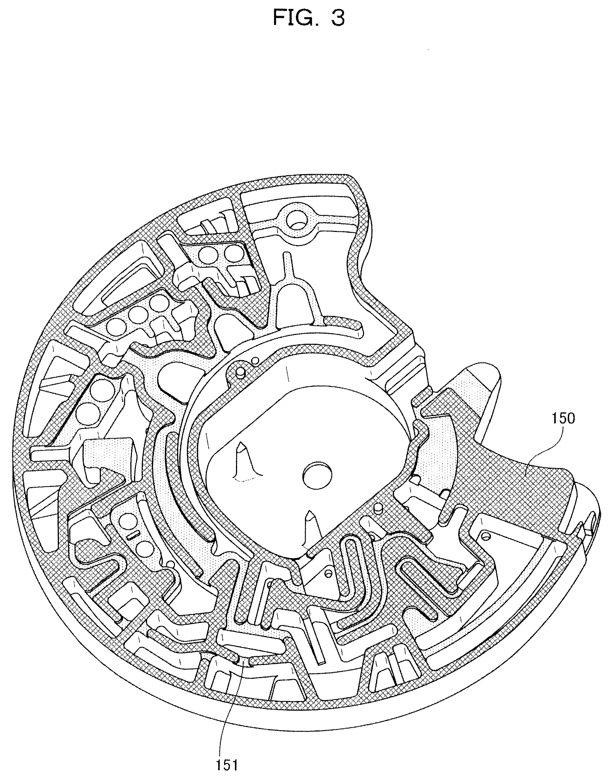

FIG. 3 illustrates the uneven surface of the base substrate 3. Hatching 150 indicates a bonded surface to the cover substrate 4. Hatching 151 indicates a point that is slightly lower than the bonded surface to the cover substrate 4 and serves as a clearance receiving a capillary force after the base substrate 3 is bonded to the cover substrate 4.

On the bottom of the analysis device 1, that is, on the cover substrate 4, a rotary support section 15 is formed that protrudes on the bottom of the analysis device 1 and acts as a centering fitting part. Moreover, a rotary support section 16 is formed on the inner periphery of the protective cap 2. In the analysis device 1 with the protective cap 2 closed, the rotary support section 16 is formed in contact with the outer periphery of the rotary support section 15. On the cover substrate 4, a projecting portion 114 is formed as a detent locking section having the proximal end connected to the rotary support section 15 and the other end extending to the outer periphery of the analysis device 1.

The base substrate 3 and the cover substrate 4 are joined to each other with the diluent container 5 or the like set in the base substrate 3 and the cover substrate 4, and the protective cap 2 is attached to the joined base substrate 3 and cover substrate 4.

The cover substrate 4 covers the openings of several recessed sections formed on the top surface of the base substrate 3, thereby forming multiple storage areas and the passages of the microchannel structure connecting the storage areas, which will be described later.

Reagents required for various analyses are carried beforehand in necessary ones of the storage areas. One side of the protective cap 2 is pivotally supported such that the protective cap 2 can be opened and closed in engagement with shafts 6a and 6b formed on the base substrate 3 and the cover substrate 4. In the case where a sample liquid to be inspected is blood, the passages of the microchannel structure receiving a capillary force each have a clearance of 50 µm to 300 µm.

The outline of an analyzing process using the analysis device 1 is that a sample liquid is dropped into the analysis device 1 containing the diluent having been set beforehand, at least a portion of the sample liquid is diluted with the diluent, and then measurement is conducted.

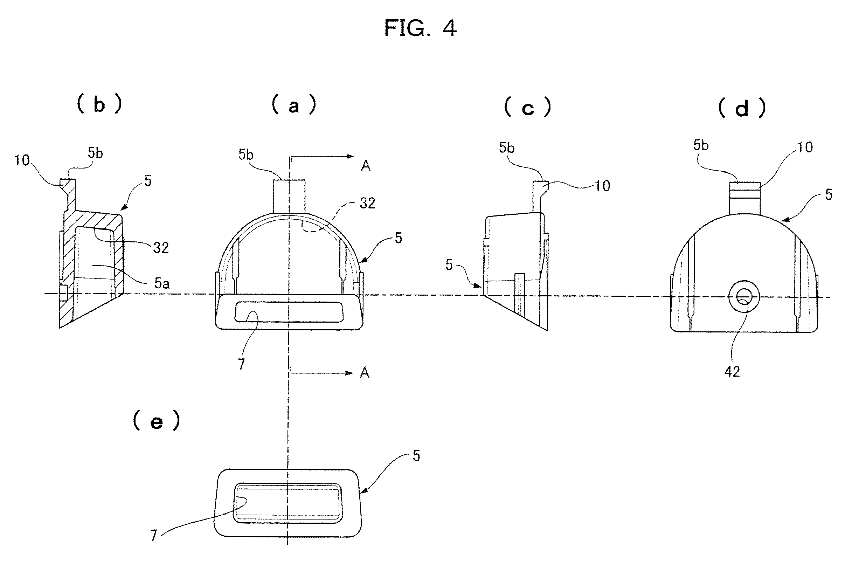

FIG. 4 illustrates the shape of the diluent container 5.

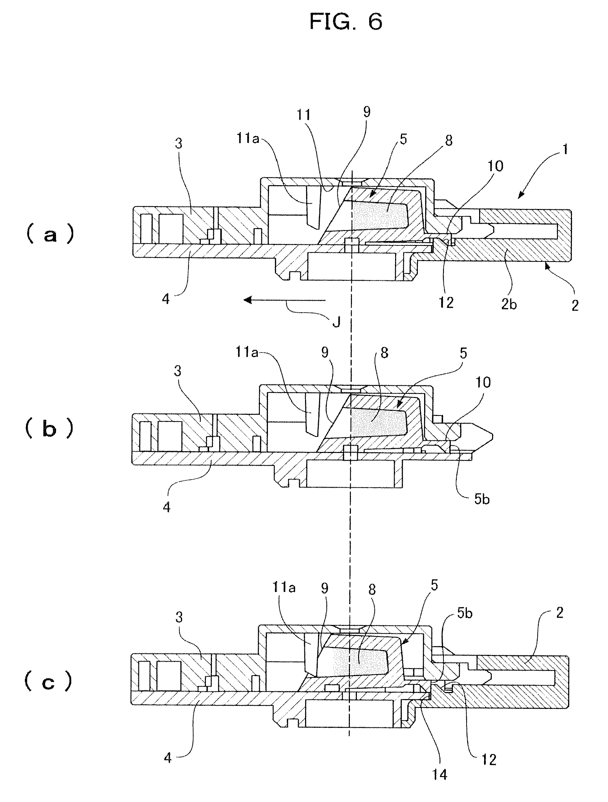

FIG. 4(a) is a plan view, FIG. 4(b) is an A-A sectional view of FIG. 4(a), FIG. 4(c) is a side view, FIG. 4(d) is a rear view, and FIG. 4(e) is a front view taken from an opening 7. An interior 5a of the diluent container 5 is filled with a diluent 8 as illustrated in FIG. 6(a), and then the opening 7 is sealed with a sealing member 9 such as aluminum foil. A latch section 10 is formed on the opposite side of the diluent container 5 from the opening 7. The diluent container 5 is set in a diluent container storage part 11 formed between the base substrate 3 and the cover substrate 4, and is accommodated movably between a liquid retaining position illustrated in FIG. 6(a) and a liquid discharging position illustrated in FIG. 6(c).

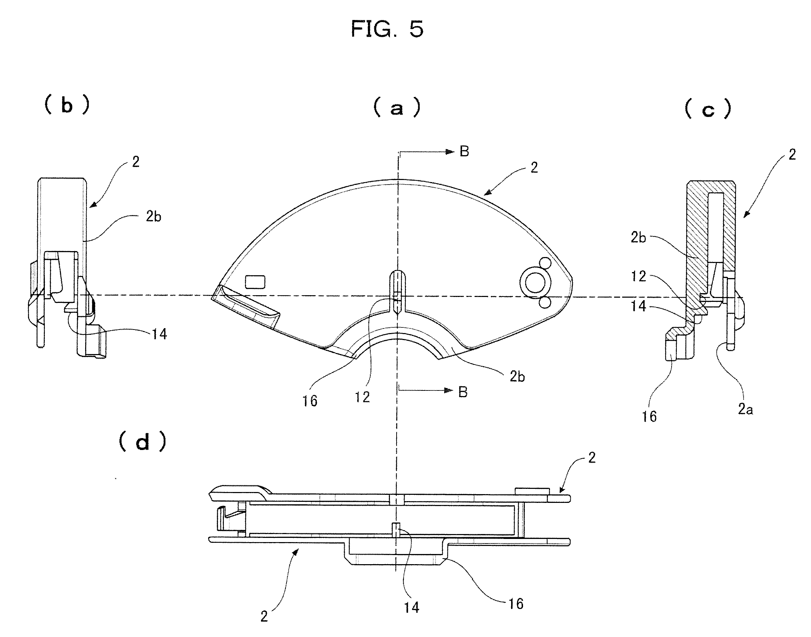

FIG. 5 illustrates the shape of the protective cap 2.

FIG. 5(a) is a plan view, FIG. 5(b) is a side view, FIG. 5(c) is a B-B sectional view of FIG. 5(a), and FIG. 5(d) is a front view taken from an opening 2a. In the protective cap 2, a locking groove 12 is formed. In the closed state of FIG. 1(a), the latch section 10 of the diluent container 5 can be engaged with the locking groove 12 as illustrated in FIG. 6(a).

FIG. 6(a) illustrates the analysis device 1 before use. In this state, the protective cap 2 is closed and the latch section 10 of the diluent container 5 is engaged with the locking groove 12 of the protective cap 2 to lock the diluent container 5 at the liquid retaining position, so that the diluent container 5 does not move in the direction of arrow J. The analysis device 1 in this state is supplied to a user.

When the sample liquid is dropped, the protective cap 2 is opened as illustrated in FIG. 1(b) against the engagement with the latch section 10 in FIG. 6(a). At this point, a bottom 2b of the protective cap 2 is elastically deformed with the locking groove 12 formed on the bottom 2b, thereby disengaging the latch section 10 of the diluent container 5 from the locking groove 12 of the protective cap 2 as illustrated in FIG. 6(b).

In this state, the sample liquid is dropped to an exposed inlet 13 of the analysis device 1 and then the protective cap 2 is closed. At this point, by closing the protective cap 2, a wall surface 14 forming the locking groove 12 comes into contact with a surface 5b of the latch section 10 of the diluent container 5 on the protective cap 2, and then the wall surface 14 presses the diluent container 5 in the direction of arrow J (a direction that comes close to the liquid discharging position). The diluent container storage part 11 has an opening rib 11a formed as a section projecting from the base substrate 3. When the diluent container 5 is pressed by the protective cap 2, the sealing member 9 provided on the inclined seal face of the opening 7 of the diluent container 5 is collided with and broken by the opening rib 11a as illustrated in FIG. 6(c).

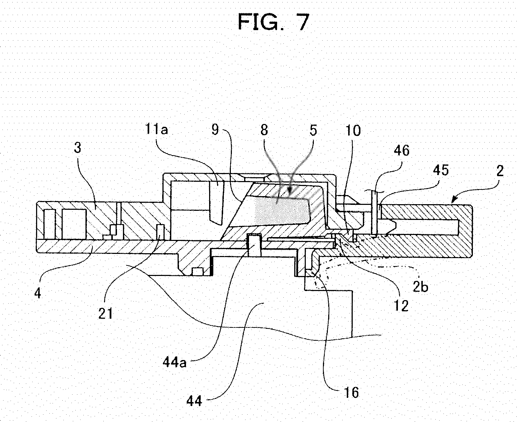

FIG. 7 illustrates a manufacturing process in which the analysis device 1 is set at the shipment state of FIG. 6(a). First, before the protective cap 2 is closed, a groove 42 (see FIGS. 2 and 4(d)) provided on the undersurface of the diluent container 5 and a hole 43 provided on the cover substrate 4 are aligned with each other, and a projecting portion 44a of a locking member 44 is engaged with the groove 42 of the diluent container 5 through the hole 43 at the liquid retaining position. The projecting portion 44a is provided separately from the base substrate 3 or the cover substrate 4. The diluent container 5 is set so as to be locked at the liquid retaining position. Further, from a notch 45 (see FIG. 1) formed on the top surface of the protective cap 2, a pressing member 46 is inserted to press the bottom of the protective cap 2, so that the protective cap 2 is elastically deformed. In this state, the protective cap 2 is closed and then the pressing member 46 is removed, so that the analysis device 1 can be set in the state of FIG. 6(a).

The present embodiment described an example in which the groove 42 is provided on the undersurface of the diluent container 5. The groove 42 may be provided on the top surface of the diluent container 5 and the hole 43 may be provided on the base substrate 3 in alignment with the groove 42 such that the projecting portion 44a of the locking member 44 is engaged with the groove 42.

Furthermore, the locking groove 12 of the protective cap 2 is directly engaged with the latch section 10 of the diluent container 5 to lock the diluent container 5 at the liquid retaining position. The locking groove 12 of the protective cap 2 and the latch section 10 of the diluent container 5 may be indirectly engaged with each other to lock the diluent container 5 at the liquid retaining position.

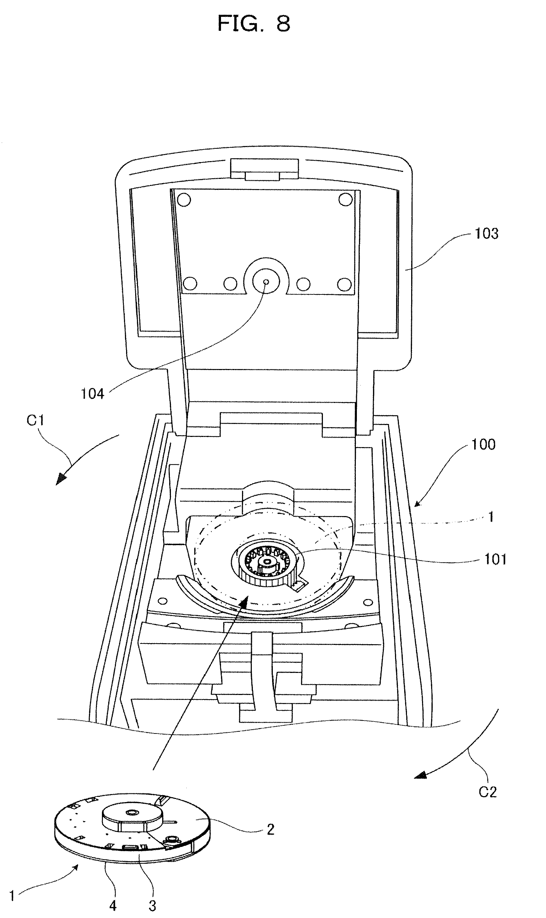

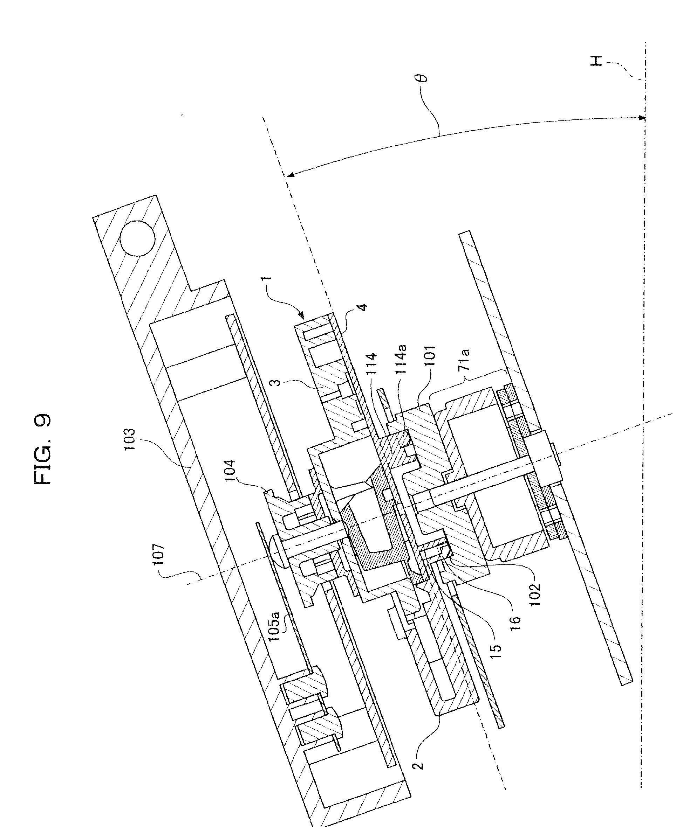

As illustrated in FIGS. 8 and 9, the analysis device 1 is set on a turntable 101 of an analyzing apparatus 100.

In the present embodiment, the turntable 101 is attached around a rotation axis 107 tilted as illustrated in FIG. 9 and is tilted by angle θ (10° to 45°) with respect to horizontal line H. The direction of gravity applied to a solution in the analysis device 1 can be controlled according to the rotation stop position of the analysis device 1.

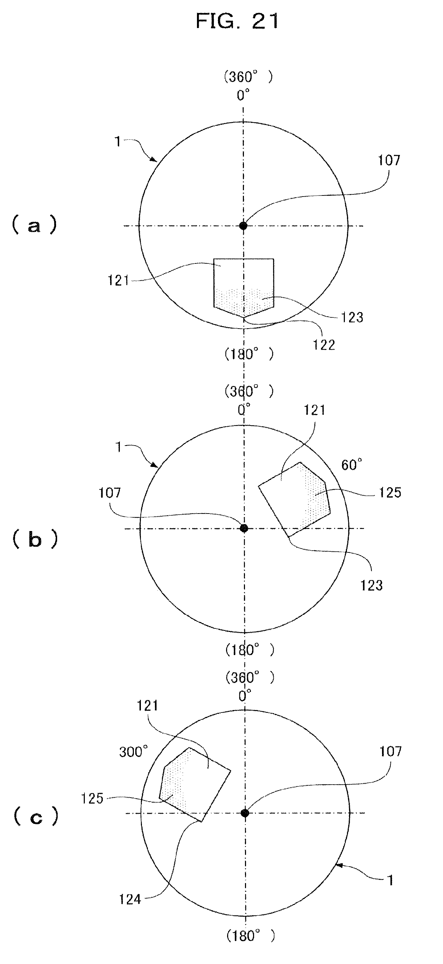

To be specific, in the case where the analysis device 1 is stopped at the position of FIG. 21(a) (a position around 180° when a position directly above the analysis device 1 in FIG. 21(a) is represented as 0° (360°)), an underside 122 of an operation cavity 121 is directed downward when viewed from the front. Thus, a force of gravity to a solution 125 in the operation cavity 121 is applied toward the outer periphery (underside 122) of the analysis device 1.

In the case where the analysis device 1 is stopped at a position around 60° as illustrated in FIG. 21(b), an upper left side 123 of the operation cavity 121 is directed downward when viewed from the front. Thus, a force of gravity is applied to the upper left of the solution 125 in the operation cavity 121. Likewise, at a position around 300° in FIG. 21(c), an upper right side 124 of the operation cavity 121 is directed downward when viewed from the front. Thus, a force of gravity is applied to the upper right of the solution 125 in the operation cavity 121.

In this way, the rotation axis 107 is tilted and the analysis device 1 is stopped at any position, so that a driving force can be used for transferring a solution in the analysis device 1 in a predetermined direction.

A force of gravity to a solution in the analysis device 1 can be set by adjusting the angle θ of the rotation axis 107, desirably depending on the relationship between a quantity of transferred liquid and the adhesion of applied liquid on a wall surface in the analysis device 1.

In the case where the angle θ is smaller than 10°, a force of gravity applied to the solution is so small that a driving force for transfer may not be obtained. In the case where the angle 0 is larger than 45°, a load applied to the rotation axis 107 may increase or the solution transferred by a centrifugal force may unexpectedly move under its own weight and lead to an uncontrollable state.

A circular groove 102 is formed on the top surface of the turntable 101. In a state in which the analysis device 1 is set on the turntable 101, the rotary support section 15 formed on the cover substrate 4 of the analysis device 1 and the rotary support section 16 formed on the protective cap 2 are engaged with the circular groove 102 to accommodate the analysis device 1.

After the analysis device 1 is set on the turntable 101, a door 103 of the analyzing apparatus is closed before a rotation of the turntable 101, so that the set analysis device 1 is pressed to the turntable 101 by a clamper 104 provided on the door 103, at a position on the rotation axis of the turntable 101 by a biasing force of a spring 105a that serves as a biasing member. The analysis device 1 rotates with the turntable 101 that is rotationally driven by a brushless motor 71a of a rotational drive unit 106. Reference numeral 107 denotes the rotation axis of the turntable 101.

With this configuration, when the analysis device 1 is set on the turntable 101, as illustrated in FIG. 9, an end 114a of the projecting portion 114 of the analysis device 1 is engaged with any one of grooves formed at regular intervals on the inner periphery of the circular groove 102 of the turntable 101, so that the analysis device 1 does not slip in the circumferential direction of the turntable 101.

The protective cap 2 is attached to prevent the sample liquid applied around the inlet 13 from being splashed to the outside by a centrifugal force during analysis.

The components constituting the analysis device 1 are desirably made of resin materials enabling low material cost with high mass productivity. The analyzing apparatus 100 analyzes the sample liquid according to an optical measurement method for measuring light having passed through the analysis device 1. Thus, the base substrate 3 and the cover substrate 4 are desirably made of transparent synthetic resins including PC, PMMA, AS, and MS.

The diluent container 5 is desirably made of crystalline synthetic resins such as PP and PE that have low moisture permeability. This is because the diluent container 5 has to contain the diluent 8 for a long time period. The protective cap 2 may be made of any materials as long as high moldability is obtained. Inexpensive resins such as PP, PE, and ABS are desirable.

The base substrate 3 and the cover substrate 4 are desirably joined to each other according to a method hardly affecting the reaction activity of a reagent retained in the storage area. Thus, methods such as ultrasonic welding and laser welding are desirable by which a reactive gas and a solvent are hardly generated during joining.

On a part where a solution is transferred by a capillary force in a small clearance between the base substrate 3 and the cover substrate 4 that are joined to each other, hydrophilic treatment is performed to increase the capillary force. To be specific, hydrophilic treatment is performed using a hydrophilic polymer, a surface-active agent, and so on. In this case, hydrophilicity is a state in which a contact angle is less than 90° relative to water. More preferably, the contact angle is less than 40°.

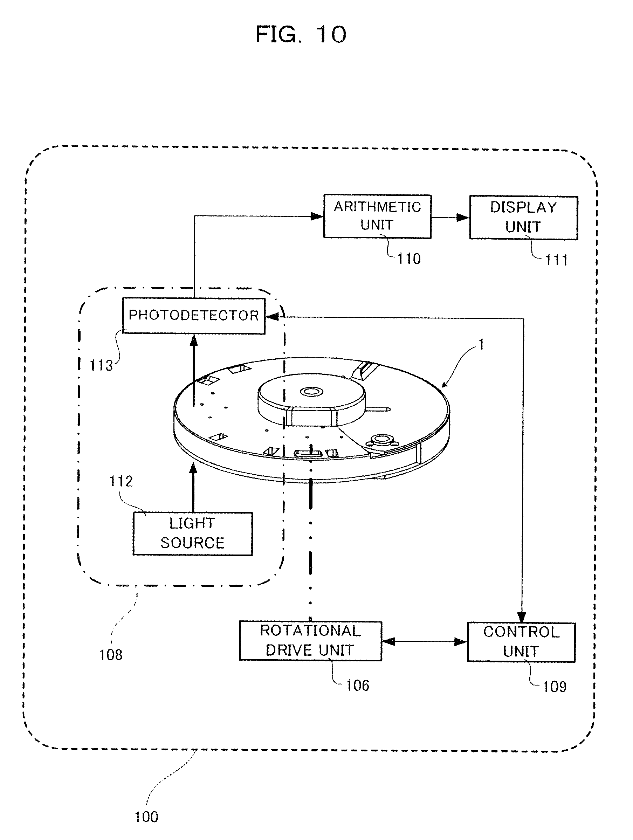

FIG. 10 shows the configuration of the analyzing apparatus 100.

The analyzing apparatus 100 includes the rotational drive unit 106 for rotating the turntable 101, an optical measurement unit 108 for optically measuring a solution in the analysis device 1, a control unit 109 for controlling, e.g., the rotation speed and direction of the turntable 101 and the measurement timing of the optical measurement unit, an arithmetic unit 110 for calculating a measurement result by processing a signal obtained by the optical measurement unit 108, and a display unit 111 for displaying the result obtained by the arithmetic unit 110.

The rotational drive unit 106 can rotate the analysis device 1 through the turntable 101 about the rotation axis 107 in any direction at a predetermined rotation speed and can further oscillate the analysis device 1 such that the analysis device 1 laterally reciprocates at a predetermined stop position with respect to the rotation axis 107 with a predetermined amplitude range and a predetermined period.

The optical measurement unit 108 includes a light source 112 for emitting light of a specific wavelength to the measurement section of the analysis device 1, and a photodetector 113 for detecting the quantity of light having passed through the analysis device 1 out of the light emitted from the light source 112.

The analysis device 1 is rotationally driven by the turntable 101, and then the sample liquid dropped into the analysis device 1 from the inlet 13 is transferred in the analysis device 1 by a centrifugal force generated by rotating the analysis device 1 about the rotation axis 107 located inside the inlet 13 and the capillary force of a capillary passage provided in the analysis device 1. The microchannel structure of the analysis device 1 will be specifically described below along with an analyzing process.

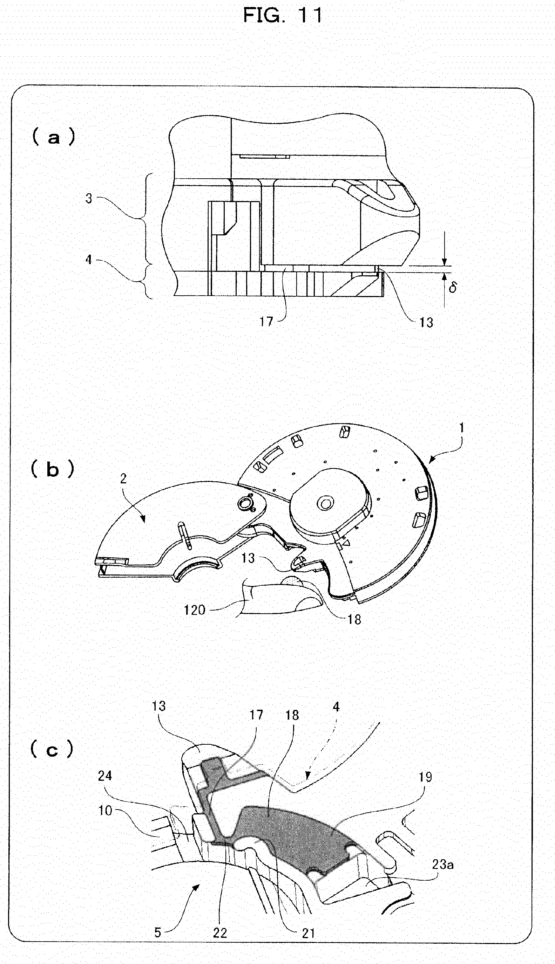

FIG. 11 illustrates a part around the inlet 13 of the analysis device 1.

FIG. 11(a) is an enlarged view of the inlet 13 viewed from the outside of the analysis device 1. FIG. 11(b) shows that the protective cap 2 is opened to collect a sample liquid 18 from a fingertip 120. FIG. 11(c) illustrates the microchannel structure viewed from the turntable 101 through the cover substrate 4.

The inlet 13 projects to the outer periphery of the analysis device 1 from the rotation axis 107 set in the analysis device 1. Moreover, the inlet 13 is connected to a capillary cavity 19 through a guide section 17 receiving a capillary force with a small clearance δ that is formed between the base substrate 3 and the cover substrate 4 so as to extend to the inner periphery of the analysis device 1. The capillary cavity 19 can retain a required quantity of the sample liquid 18 by a capillary force. The protective cap 2 is opened to directly apply the sample liquid 18 into the inlet 13, so that the sample liquid applied around the inlet 13 is drawn into the analysis device 1 by the capillary force of the guide section 17.

A bending section 22 is formed on the guide section 17, the capillary cavity 19, and the connected section. The bending section 22 including a recessed section 21 on the base substrate 3 changes the direction of a passage.

When viewed from the guide section 17, a receiving cavity 23a is formed behind the capillary cavity 19. The receiving cavity 23a has a clearance in which a capillary force is not applied. A cavity 24 opened to the atmosphere is formed partially on the sides of the capillary cavity 19, the bending section 22, and the guide section 17. The effect of the cavity 24 allows the sample liquid collected from the inlet 13 to pass through the guide section 17 and preferentially flows along the side walls of the capillary cavity 19 while avoiding the cavity 24. Thus, in the case where air bubbles are entrained from the inlet 13, the air is discharged to the cavity 24 in a section where the guide section 17 is adjacent to the cavity 24, so that the sample liquid 18 can be collected without entraining air bubbles.

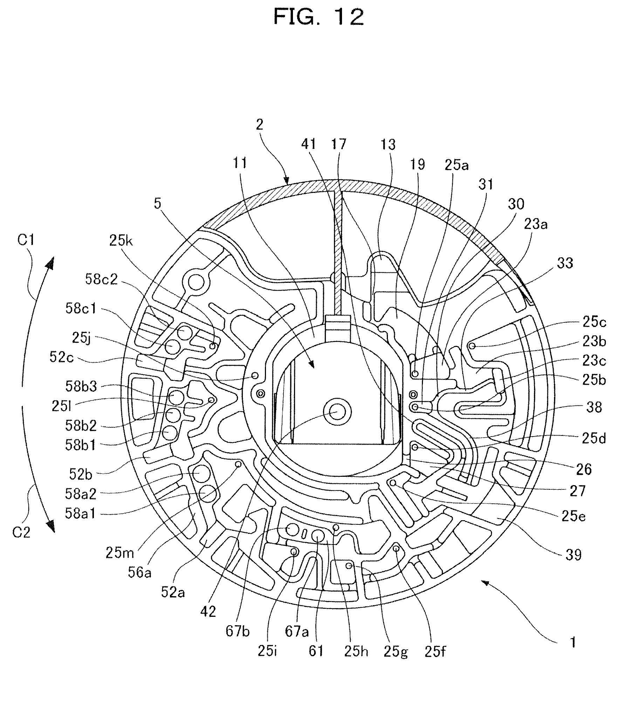

FIG. 12 illustrates a state before the analysis device 1 containing the dropped sample liquid 18 is set on the turntable 101 and is rotated thereon. At this point, as illustrated in FIG. 6(c), the sealing member 9 of the diluent container 5 has been collided with and broken by the opening rib 11a. Reference characters 25a to 25m denote air holes formed on the base substrate 3.

The following will describe the analyzing process along with the configuration of the control unit 109 that controls the operation of the rotational drive unit 106.

### - Step 1 -

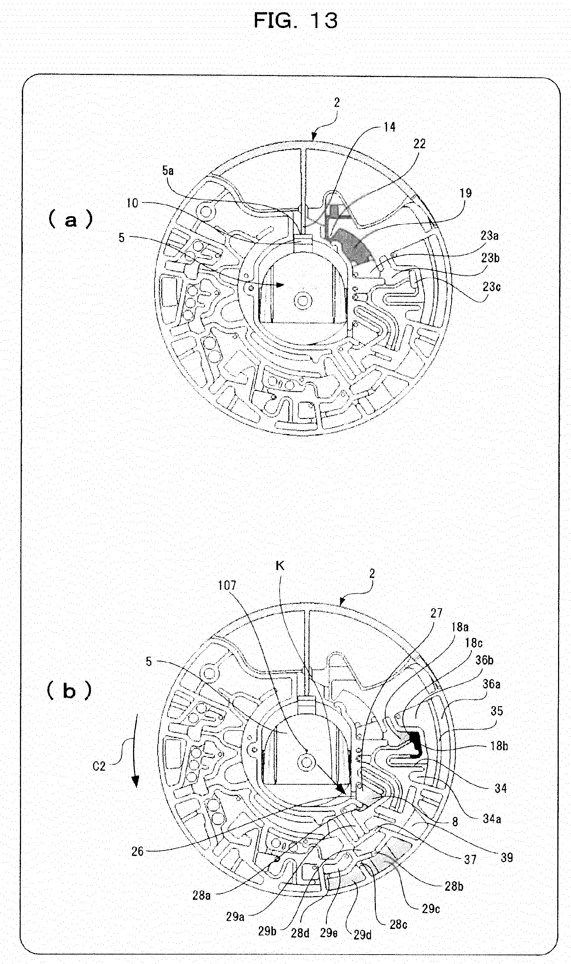

The analysis device 1 in which a sample liquid to be inspected has been dropped into the inlet 13 is set on the turntable 101. As illustrated in FIG. 13(a), the sample liquid is retained in the capillary cavity 19 and the sealing member 9 of the diluent container 5 has been broken.

### - Step 2 -

The door 103 is closed and then the turntable 101 is rotationally driven (5000 rpm to 8000 rpm) in a clockwise direction (direction C2), so that the retained sample liquid overflows at the position of the bending section 22. The sample liquid in the guide section 17 is discharged into the protective cap 2. After that, as illustrated in FIG. 13(b), the sample liquid 18 in the capillary cavity 19 flows into separating cavities 23b and 23c through the receiving cavity 23a. The analysis device 1 is rotated for 40 to 70 seconds, so that the sample liquid 18 is centrifugally separated into a plasma component 18a and a blood cell component 18b by the separating cavities 23b and 23c.

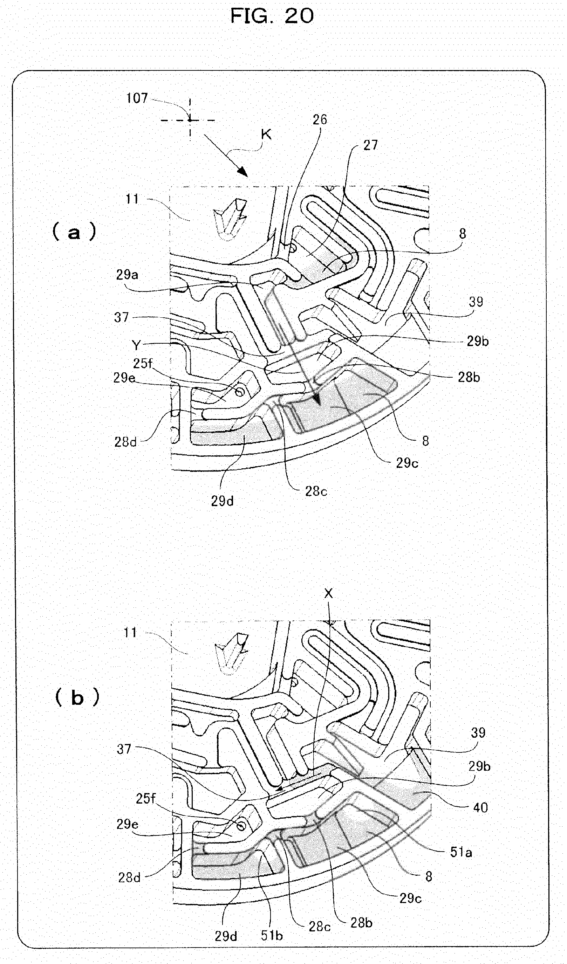

As indicated by arrow K in FIGS. 13(b) and 20(a), the diluent 8 from the diluent container 5 flows into a reserving cavity 27 through a discharging passage 26. When the diluent 8 having flowed into the reserving cavity 27 exceeds a predetermined quantity, an excessive quantity of the diluent 8 flows into an overflow cavity 29a through an overflow passage 28a, passes over a capillary passage 37 as indicated by arrow Y, and flows into an overflow cavity 29c, which serves as a reference measuring cell, through an overflow cavity 29b and an overflow passage 28b.

When the diluent having flowed into the overflow cavity 29c exceeds a predetermined quantity as in the reserving cavity 27, an excessive quantity of the diluent flows into an overflow cavity 29d through an overflow passage 28c.

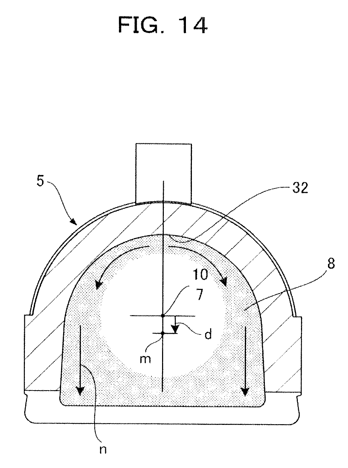

As illustrated in FIGS. 4(a) and 4(b), the bottom of the diluent container 5 on the opposite side from the opening 7 sealed with the sealing member 9 is formed of a curved surface 32. At the liquid discharging position of the diluent container 5 in the state of FIG. 13(b), a center m of the curved surface 32 is offset, as illustrated in FIG. 14, by a distance d from the rotation axis 107 to the discharging passage 26. Thus, the flow of the diluent 8 to the curved surface 32 is changed to a flow (arrow n) from the outside to the opening 7 along the curved surface 32, and then the diluent 8 is efficiently discharged to the diluent container storage part 11 from the opening 7 of the diluent container 5.

### - Step 3 -

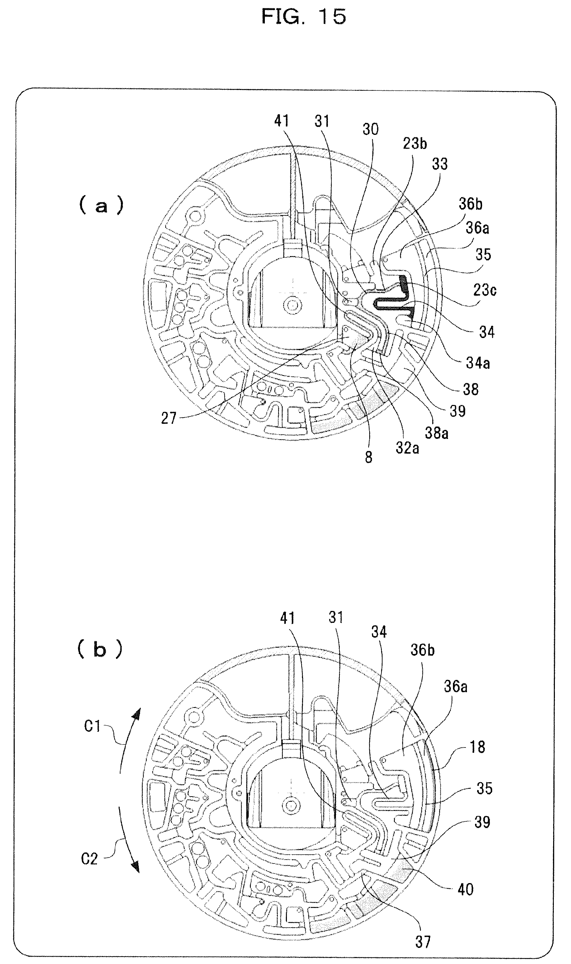

Next, when the rotation of the turntable 101 is stopped, the plasma component 18a is sucked into a capillary cavity 33 formed on the wall surface of the separating cavity 23b and flows, as illustrated in FIG. 15(a), into a measuring passage 38 through a connecting passage 30 communicating with the capillary cavity 33, so that a fixed quantity of the plasma component 18a is retained.

In the present embodiment, a filling confirming area 38a is formed at the outlet of the measuring passage 38 so as to extend to the inner periphery of the analysis device 1. Before advancing to the subsequent process, the analysis device 1 is slowly rotated at around 100 rpm and the presence or absence of the plasma component 18a can be optically detected in a state in which the filling confirming area 38a retains the plasma component 18a. The filling confirming area 38a in the analysis device 1 has a rough inner surface that scatters light passing through the filling confirming area 38a. In the case where the filling confirming area 38a is not filled with the plasma component 18a, the quantity of transmitted light decreases. In the case where the filling confirming area 38a is filled with the plasma component 18a, the liquid is also applied to the minutely uneven surface, so that the scattering of light is suppressed to increase the quantity of transmitted light. The presence or absence of the plasma component 18a can be detected by detecting a difference in light quantity.

The sample liquid in the separating cavities 23b and 23c is sucked into a siphon-shaped connecting passage 34 that connects the separating cavity 23c and an overflow cavity 36b. The diluent 8 is similarly sucked into a siphon-shaped connecting passage 41 that connects the reserving cavity 27 and a mixing cavity 39.

In this configuration, a flow preventing groove 32a at the outlet of the connecting passage 41 is formed to prevent the diluent 8 from flowing from the connecting passage 41 into the measuring passage 38. A flow preventing groove 32a is formed with a depth of about 0.2 mm to 0.5 mm on the base substrate 3 and the cover substrate 4.

The capillary cavity 33 is formed from the outermost position of the separating cavity 23b to the inner periphery of the analysis device 1. In other words, the outermost position of the capillary cavity 33 is extended outside a separation interface 18c of the plasma component 18a and the blood cell component 18b in FIG. 13(b).

By setting the position of the outer periphery of the capillary cavity 33 in this way, the outer end of the capillary cavity 33 is immersed in the plasma component 18a and the blood cell component 18b that have been separated in the separating cavity 23b. The plasma component 18a has a lower viscosity than the blood cell component 18b, so that the plasma component 18a is preferentially sucked by the capillary cavity 33. The plasma component 18a can be transferred to the measuring passage 38 through the connecting passage 30.

After the plasma component 18a is sucked, the blood cell component 18b is also sucked following the diluted plasma component 18a. Thus, the plasma component 18a can be replaced with the blood cell component 18b in the capillary cavity 33 and a path halfway to the connecting passage 30. When the measuring passage 38 is filled with the plasma component 18a, the transfer of the liquid is stopped also in the connecting passage 30 and the capillary cavity 33, so that the blood cell component 18b does not enter the measuring passage 38.

### - Step 4 -

When the turntable 101 is rotationally driven (4000 rpm to 6000 rpm) in the clockwise direction (direction C2), as illustrated in FIG. 15(b), the plasma component 18a retained in the measuring passage 38 overflows at the position of an opened-to-atmosphere cavity 31 and only a fixed quantity of the plasma component 18a flows into the mixing cavity 39. The diluent 8 in the reserving cavity 27 also flows into the mixing cavity 39 through the siphon-shaped connecting passage 41.

The sample liquid 18 in the separating cavities 23b and 23c, the connecting passage 30, and the capillary cavity 33 flows into an overflow cavity 36a through the siphon-shaped connecting passage 34 and a backflow preventing passage 35.

### - Step 5 -

Next, the rotation of the turntable 101 is stopped, the analysis device 1 is set at the position of FIG. 15(b), and the turntable 101 is controlled at a frequency of 20 Hz to 70 Hz so as to oscillate the analysis device 1 by about ±1 mm, thereby agitating the diluent 8 transferred into the mixing cavity 39 and diluted plasma 40 to be measured, the diluted plasma 40 containing the plasma component 18a.

### - Step 6 -

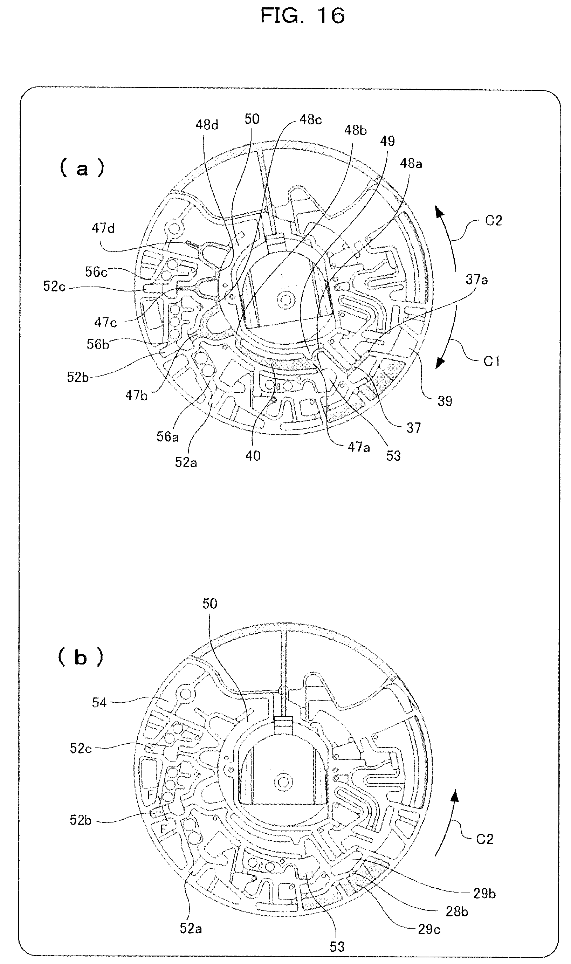

After that, the analysis device 1 is set at the position of FIG. 16(a), the oscillation of the turntable 101 is gradually increased to about 100 Hz so as to oscillate the analysis device 1 by about ±1 mm, so that the diluted plasma 40 retained in the mixing cavity 39 is transferred to the inlet of the capillary passage 37 formed inside the liquid level of the diluted plasma 40.

The diluted plasma 40 transferred to the inlet of the capillary passage 37 is sucked into the capillary passage 37 by a capillary force as indicated by arrow X and then is transferred sequentially to the capillary passage 37, measuring passages 47a, 47b, and 47c, and an overflow passage 47d.

### - Step 7 -

When the turntable 101 is rotationally driven (4000 rpm to 6000 rpm) in the clockwise direction (direction C2), as illustrated in FIG. 16(b), the diluted plasma 40 retained in the measuring passages 47a, 47b, and 47c overflows at the positions of bending sections 48a, 48b, 48c, and 48d that are connected to an opened-to-atmosphere cavity 50 communicating with the atmosphere, and then only a fixed quantity of the diluted plasma 40 flows into measuring cells 52b and 52c and a reserving cavity 53.

The diluted plasma 40 retained in the overflow passage 47d at this point flows into an overflow cavity 54 through a backflow preventing passage 55. The diluted plasma 40 in the capillary passage 37 at this point flows into the overflow cavity 29c through the overflow cavity 29b and the overflow passage 28b.

On a part of the side wall of the measuring passage 47a, a recessed section 49 is formed near the bending section 48a so as to communicate with the opened-to-atmosphere cavity 50. Thus, the adhesion of liquid on the wall surface decreases near the bending section 48a so that the liquid is drained well at the bending section 48a.

Measuring cells 52a, 52b and 52c are extended in a direction along which a centrifugal force is applied. To be specific, the measuring cells are extended from the center of rotation to the outermost periphery of the analysis device 1 so as to decrease in width in the circumferential direction of the analysis device 1.

The bottoms of the outer peripheries of the multiple measuring cells 52a to 52c are disposed at the same radius of the analysis device 1. Thus, for measurements in the multiple measuring cells 52a to 52c, it is not necessary to provide multiple light sources 112 of the same wavelength or multiple photodetectors 113 at different radius distances for the respective light sources 112, thereby reducing the cost of the apparatus. Since measurement can be conducted using different wavelengths in the same measurement cell, the sensitivity of measurement can be improved by selecting the optimum wavelength according to the concentration of a mixed solution.

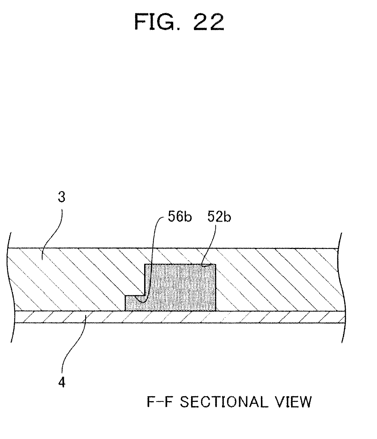

On one side walls of the measuring cells 52a to 52c in the circumferential direction, capillary areas 56a to 56c are formed that carry reagents so as to extend from the outer periphery positions to the inner peripheries of the measuring cells. FIG. 22 is an F-F sectional view of FIG. 16(b).

The suction capacity of the capillary area 56b is not so large as to fully accommodate the sample liquid retained in the measuring cell 52b. Similarly, the capacities of the capillary areas 56a and 56c are not so large as to fully accommodate the sample liquid retained in the measuring cells 52a and 52c.

The optical path lengths of the measuring cells 52a to 52c are adjusted according to the range of absorbance obtained from a mixed solution after a reaction of a component to be tested and reagents.

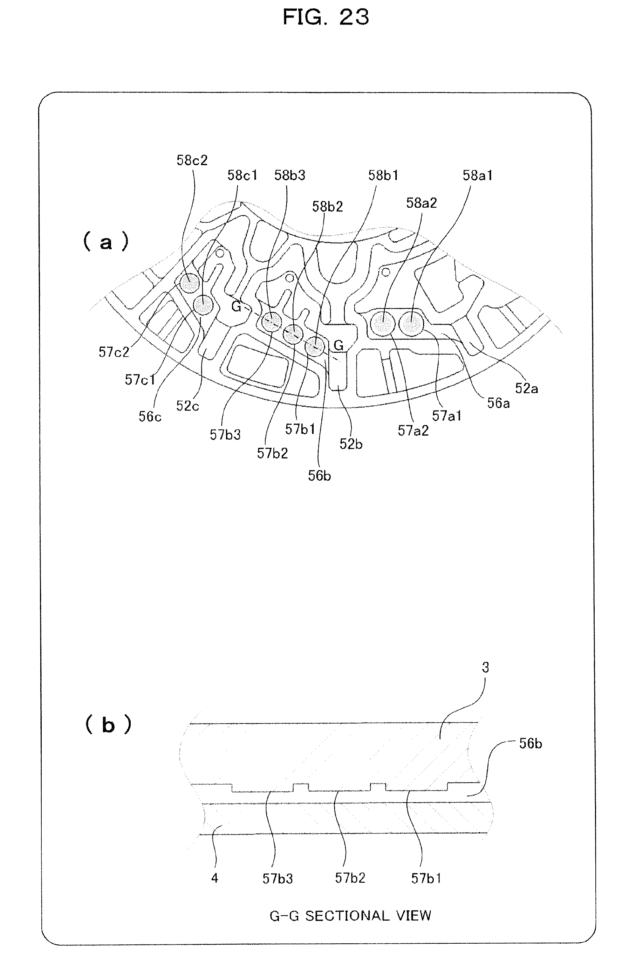

In the capillary areas 56a, 56b, and 56c, as illustrated in FIG. 23(a), reagents 58a1, 58a2, 58b1, 58b2, 58b3, 58c1, and 58c2 to be reacted with a component to be tested are respectively contained in reagent carrying sections 57a1, 57a2, 57b1, 57b2, 57b3, 57c1, and 57c2 formed in the capillary areas 56a, 56b, and 56c. FIG. 23(b) is a G-G sectional view of FIG. 23(a).

The reagent carrying sections 57b1, 57b2, and 57b3 are protruded from the capillary area 56b such that a clearance between the reagent carrying sections 57b1, 57b2, and 57b3 and the cover substrate 4 is smaller than a clearance between the capillary area 56b and the cover substrate 4.

The reagents 58b1, 58b2, and 58b3 are applied to the reagent carrying sections 57b1, 57b2, and 57b3, so that the expansion of the reagents 58b1, 58b2, and 58b3 can be suppressed by steps formed by the reagent carrying sections 57b1, 57b2, and 57b3 and the capillary area 56b. Thus, the different reagents can be carried without being mixed.

The clearance of the reagent carrying sections 57b1, 57b2, and 57b3 is smaller than that of the capillary area 56b and thus liquid sucked into the capillary area 56b is reliably supplied into the reagent carrying sections 57b1, 57b2, and 57b3. Consequently, the reagents 58b1, 58b2, and 58b3 can be reliably dissolved.

The capillary area 56b has a clearance of about 50 µm to 300 µm, which enables the application of a capillary force. Thus, the reagent carrying sections 57b1, 57b2, and 57b3 are protruded from the capillary area 56b only by about several tens µm. The capillary areas 56a and 56c are similarly configured.

### - Step 8 -

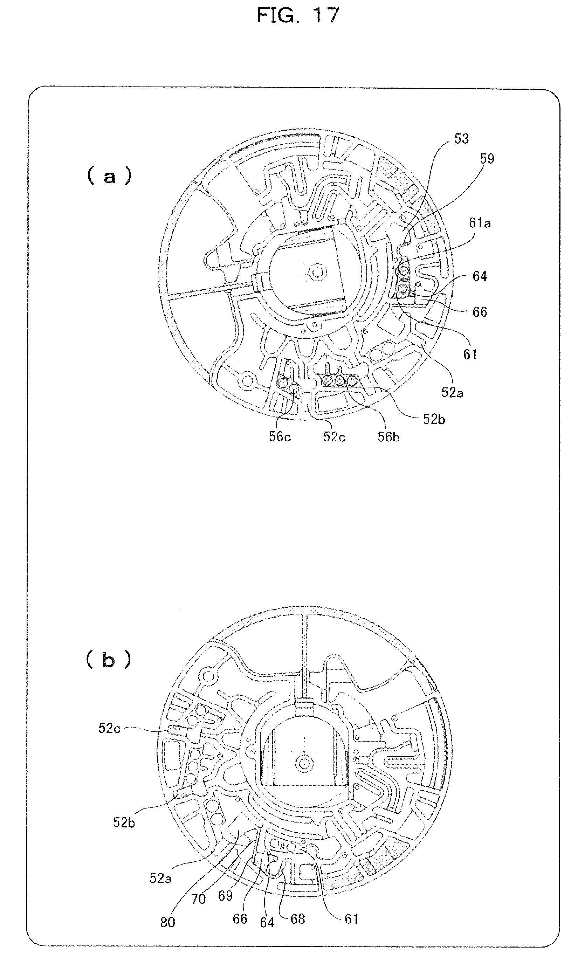

Next, the rotation of the turntable 101 is stopped, the analysis device 1 is set at the position of FIG. 17(a), and then the turntable 101 is controlled at a frequency of 60 Hz to 120 Hz so as to oscillate the analysis device 1 by about ±1 mm, so that the diluted plasma 40 retained in the reserving cavity 53 is transferred to an operation cavity 61 by the action of a capillary force through a connecting section 59. The connecting section 59 is formed on the side wall of the reserving cavity 53 so as to be immersed under the liquid level of the diluted plasma 40.

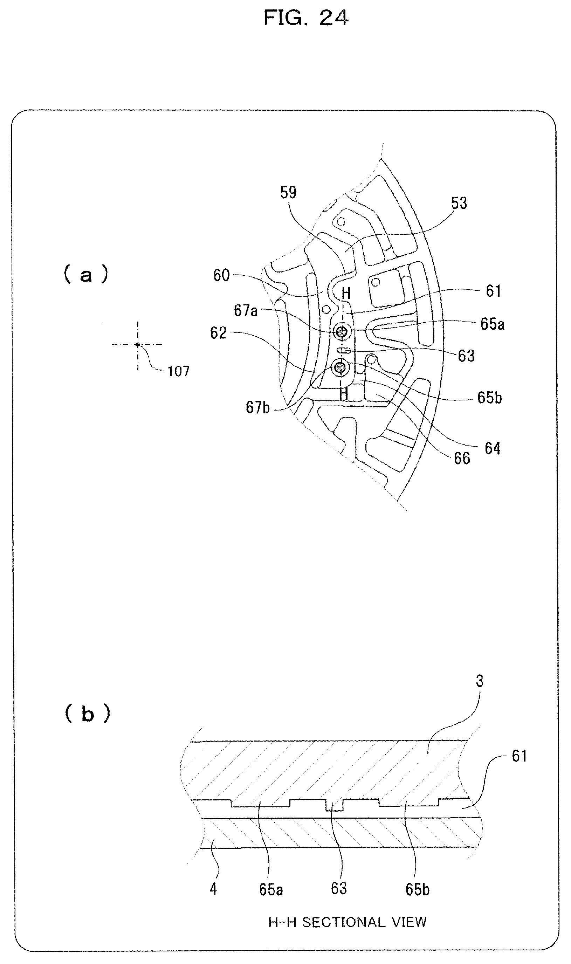

Furthermore, the turntable 101 is controlled at a frequency of 10 Hz to 40 Hz for 40 to 60 seconds to agitate reagents 67a and 67b contained in the operation cavity 61 illustrated in FIG. 24(a) and the diluted plasma 40, so that a specific component in the diluted plasma 40 is reacted with the reagents.

In this case, HDL cholesterol is to be measured in the measuring cell 52a. The reagents 67a and 67b carried in the operation cavity 61 in a dry state are HDL cholesterol analyzing reagents that coagulate and precipitate non-HDL components that are unnecessary for analysis. Specifically, sodium tungstophosphate (NACALAI TESQUE, INC.) was used.

The diluted plasma 40 transferred to the measuring cells 52b and 52c is, as illustrated in FIG. 17(a), sucked into the capillary areas 56b and 56c by a capillary force. At this point, the reagents 58b1, 58b2, 58b3, 58c1, and 58c2 start dissolving and the specific component in the diluted plasma 40 starts reacting with the reagents.

As illustrated in FIG. 24(a), the operation cavity 61 is formed next to the reserving cavity 53 in the circumferential direction with respect to the rotation axis 107. A clearance of the operation cavity 61 from the cover substrate 4 enables the application of a capillary force, and the reagents 67a and 67b are carried in reagent carrying sections 65a and 65b. In the operation cavity 61, an agitating rib 63 is formed around the reagents 67a and 67b, to be specific, between the reagents 67a and 67b. The agitating rib 63 is extended in the radial direction and is lower in height than the dimension of the external wall of the operation cavity 61 (= the clearance between the base substrate 3 and the cover substrate 4).

As illustrated in FIG. 24(b), the cross sectional dimension of the agitating rib 63 in the thickness direction of the cover substrate 4 is smaller than the cross sectional dimension of the operation cavity 61 in the thickness direction of the cover substrate 4. In other words, the reagent carrying sections 65a and 65b are protruded from the operation cavity 61 such that the clearance of the reagent carrying sections 65a and 65b is smaller than that of the operation cavity 61.

The reagent carrying sections 65a and 65b are protruded from the operation cavity 61 such that a clearance between the reagent carrying sections 65a and 65b and the cover substrate 4 is smaller than that between the operation cavity 61 and the cover substrate 4.

Since the clearance of the reagent carrying sections 65a and 65b is smaller than that of the operation cavity 61, liquid flowing into the operation cavity 61 is reliably supplied to the reagent carrying sections 65a and 65b. Thus, the reagents 67a and 67b can be reliably dissolved. The reagent carrying sections 65a and 65b are protruded from the operation cavity 61 only by about several tens µm.

On the inner periphery side of the operation cavity 61, a cavity 62 is formed that is connected to the reserving cavity 53 via a communicating section 60. The clearance of the cavity 62 from the cover substrate 4 does not enable the application of a capillary force. Furthermore, the cavity 62 communicates with the atmosphere through an air hole 25h formed near the communicating section 60.

The reserving cavity 53 and the operation cavity 61 are connected via the connecting section 59 that is extended from the side wall of the reserving cavity 53 through the communicating section 60. The clearance of the connecting section 59 from the cover substrate 4 enables the application of a capillary force. In this configuration, the end of the connecting section 59 is circumferentially extended beyond the liquid level of the diluted plasma 40 contained in the reserving cavity 53, with respect to the rotation axis.

On the outer periphery of the operation cavity 61, a separating cavity 66 is formed that is connected to the operation cavity 61 via a connecting passage 64. The cross sectional dimension of the connecting passage 64 from the cover substrate 4 in the thickness direction forms a clearance that enables the application of a capillary force. The cross sectional dimension is regulated so as to have a larger capillary force than that of the operation cavity 61.

Although the space of the operation cavity 61 filled with the diluted plasma 40 is as large as the clearance, a small space 61a is left without being filled with the diluted plasma 40.

In the state of FIG. 17(a), the diluted plasma 40 comes into contact with the reagents 67a and 67b and then the reagents 67a and 67b dissolve in the diluted plasma 40. In this state, the analysis device 1 is oscillated by a predetermined angle with respect to the rotation axis 107, so that the diluted plasma 40 in the operation cavity 61 is moved in the operation cavity 61 by the space 61a and is more reliably agitated by collision with the agitating rib 63 during agitation. Thus, even in the case where the reagents have high specific gravities, it is possible to effectively prevent precipitation of the reagents.

### - Step 9 -

Next, the turntable 101 is rotationally driven (5000 rpm to 7000 rpm) in the clockwise direction (direction C2), so that as illustrated in FIG. 17(b), the diluted plasma having reacted with the reagents of the operation cavity 61 passes through the connecting passage 64 and flows into the separating cavity 66. Moreover, the high-speed rotation is kept for 20 to 40 seconds, so that diluted plasma components are centrifugally separated. The diluted plasma components include non-HDL coagulated components and HDL components that have been generated in the operation cavity 61.

In the present embodiment, in a reaction of a component to be inspected and the reagents, a component inhibiting the reaction is removed in an upstream process. The diluted plasma is reacted with the reagents in the operation cavity 61, so that a specific component inhibiting a reaction in a downstream process is coagulated and then the aggregates are removed by centrifugal separation in the subsequent process.

A mixed solution of the reagents retained in the capillary areas 56b and 56c and the diluted plasma is transferred to the outer peripheries of the measuring cells 52b and 52c by a centrifugal force, so that the reagents and the diluted plasma are agitated.

In this configuration, the analysis device 1 is repeatedly rotated and stopped to accelerate the agitation of the reagents and the diluted plasma. Thus, the reagents and the diluted plasma can be reliably agitated in a short time as compared with agitation only by diffusion.

### - Step 10 -

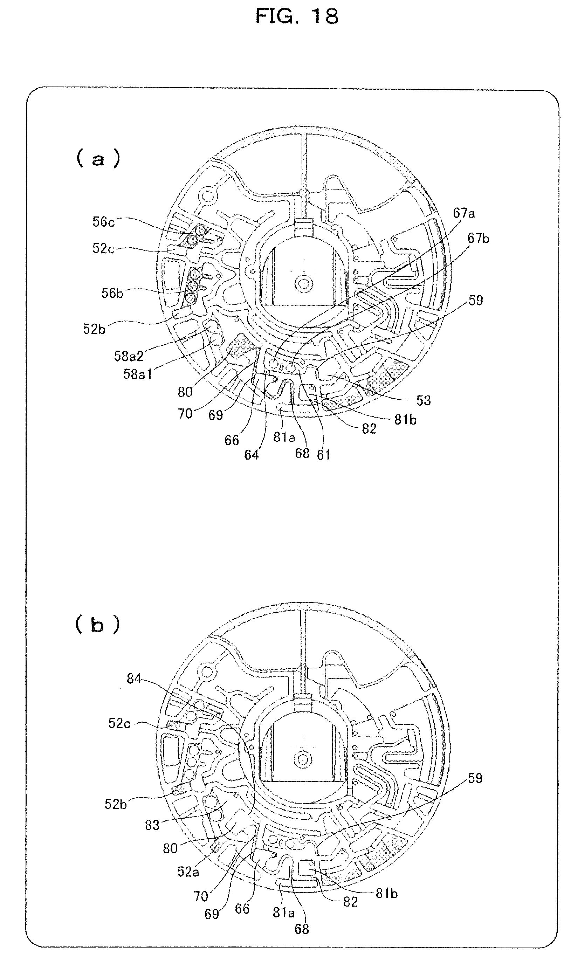

Next, when the rotation of the turntable 101 is stopped, the diluted plasma components including HDL components in the diluted plasma 40 are sucked into a capillary cavity 69 formed on the wall surface of the separating cavity 66 and flows, as illustrated in FIG. 18(a), into a measuring passage 80 through a connecting passage 70 communicating with the capillary cavity 69, so that a fixed quantity of the diluted plasma components is retained.

Moreover, the diluted plasma 40 containing the non-HDL coagulated components in the separating cavity 66 is sucked into a siphon-shaped connecting passage 68 that connects the separating cavity 66 and an overflow cavity 81a.

The mixed solution of the reagents and the diluted plasma in the measuring cells 52b and 52c is sucked into the capillary areas 56b and 56c again by a capillary force.

As illustrated in FIG. 18(a), the outermost position of the capillary cavity 69 is extended to the outer periphery of the analysis device 1 so as to be immersed in the diluted plasma retained in the separating cavity 66.

The capillary cavity 69 formed thus preferentially sucks supernatant diluted plasma rather than a precipitate having a high specific gravity, so that the diluted plasma 40 containing HDL components free from precipitates can be transferred to the measuring passage 80 through the connecting passage 70.

### - Step 11 -

When the turntable 101 is rotationally driven (4000 rpm to 6000 rpm) in the clockwise direction (direction C2), as illustrated in FIG. 18(b), the diluted plasma 40 retained in the measuring passage 80 overflows at the position of a bending section 84 that is connected to an opened-to-atmosphere cavity 83 communicating with the atmosphere, and then only a fixed quantity of the diluted plasma 40 flows into the measuring cell 52a.

The diluted plasma 40 in the separating cavity 66, the connecting passage 70, and the capillary cavity 69 flows into the overflow cavity 81a through the siphon-shaped connecting passage 68.

The mixed solution of the reagents retained in the capillary areas 56b and 56c and the diluted plasma is transferred to the outer peripheries of the measuring cells 52b and 52c by a centrifugal force, so that the reagents and the diluted plasma are agitated.

At this point, the diluted plasma 40 transferred to the overflow cavity 81a is supplied to an overflow passage 82c when the rotation of the analysis device 1 is stopped, the overflow passage 82c being connected to an overflow cavity 81b communicating with the atmosphere. Thus, the outlet of the overflow cavity 81a is sealed from the atmosphere so as to generate a negative pressure in the cavity 81a. It is therefore possible to prevent the diluted plasma 40 from passing through the connecting passage 68 from the overflow cavity 81a.

### - Step 12 -

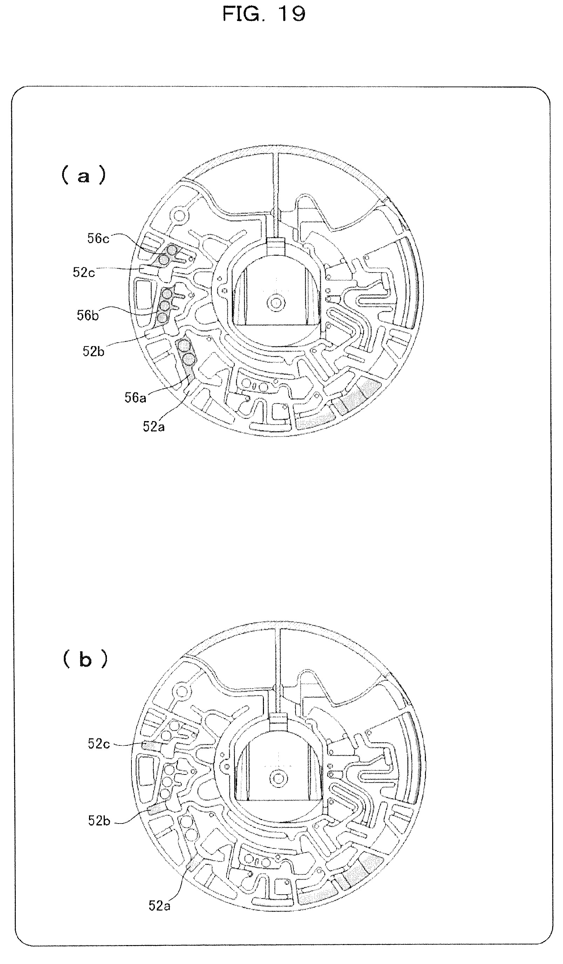

Next, when the rotation of the turntable 101 is stopped, as illustrated in FIG. 19(a), the diluted plasma 40 containing the HDL components transferred to the measuring cell 52a is sucked into the capillary area 56a by a capillary force. At this point, the reagents 58a1 and 58a2 of FIG. 23(a) start dissolving and then the specific component in the diluted plasma 40 starts reacting with the reagents.

In this case, HDL cholesterol is to be measured in the measuring cell 52a. Thus, out of the reagents 58a1 and 58a2 that are HDL measuring reagents, the reagent 58a1 carried in a dry state is an enzyme reagent. Specifically, cholesterol esterase (Toyobo Co., Ltd.), cholesterol dehydrogenase (Amano Enzyme Inc.), and diaphorase (Toyobo Co., Ltd.) were used. The reagent 58a2 carried in a dry state is a coloring reagent acting as a mediator. Specifically, NAD+ (Oriental Yeast Co., Ltd.) and WST-8 (Dojindo Laboratories) were used.

Moreover, a mixed solution of the reagents and the diluted plasma in the measuring cells 52b and 52c is sucked into the capillary areas 56b and 56c again by a capillary force.

### - Step 13 -

When the turntable 101 is rotationally driven in the clockwise direction (direction C2), as illustrated in FIG. 19(b), a mixed solution of the reagents retained in the capillary areas 56a, 56b, and 56c and the diluted plasma is transferred to the outer peripheries of the measuring cells 52a, 52b, and 52c by a centrifugal force, so that the reagents and the diluted plasma are agitated.

The operations of steps 11 and 12 are repeatedly performed for the diluted plasma 40 transferred to the measuring cell 52a, thereby accelerating the reaction of the reagents and HDL cholesterol contained in the diluted plasma. Thus, the reagents and the diluted plasma can be reliably agitated in a short time as compared with agitation only by diffusion.

### - Step 14 -

The analysis device 1 is rotationally driven (1000 rpm to 1500 rpm) in a counterclockwise direction (direction C1) or the clockwise direction (direction C2). When the measuring cells 52a, 52b, and 52c pass between the light source 112 and the photodetector 113, the arithmetic unit 110 reads a detected value of the photodetector 113 and calculates the concentration of the specific component. When the diluted plasma 40 flows into the measuring cells 52a, 52b, and 52c in steps 7 and 11, the arithmetic unit 110 reads a detected value of the photodetector 113 during the passage of the measuring cells 52a, 52b, and 52c between the light source 112 and the photodetector 113, so that an absorbance can be calculated before a reaction with the reagents. In the calculation of the arithmetic unit 110, the absorbance is used as reference data of the measuring cells 52a, 52b, and 52c, thereby improving the accuracy of measurement.

The fixed quantity of the diluted plasma 40 in the reserving cavity 53 is transferred to the measuring cell 52a by a centrifugal force and is measured while being reacted with the reagents. Thus, higher accuracy of measurement can be expected without solubility distributions of the reagents.

Furthermore, HDL components can be sequentially transferred to the reserving cavity 53, the operation cavity 61, the separating cavity 66, the measuring passage 80, the measuring cell 52a, the capillary area 56a, and the measuring cell 52a by a centrifugal force so as to efficiently reach the measuring cell 52a. Hence, the analysis device has only a small loss of the liquid sample, reducing the burden of a subject in a test.

In the present embodiment, the measuring cell is optically accessed to measure a component according to an attenuation. A component may be measured by electrically accessing the reactant of the reagent and the sample in the measuring cell. In this case, a mediator for access using an electrode may be potassium ferricyanide.

In the present embodiment, the agitating rib 63 is provided for increasing the agitation efficiency of the sample and the HDL-cholesterol analyzing reagents carried in the operation cavity 61. Similar agitating ribs may be formed in the capillary areas 56a, 56b, and 56c to increase the agitation efficiency of the sample and the reagents.

### (Second Embodiment)

Reagents in the first embodiment will be specifically described below.

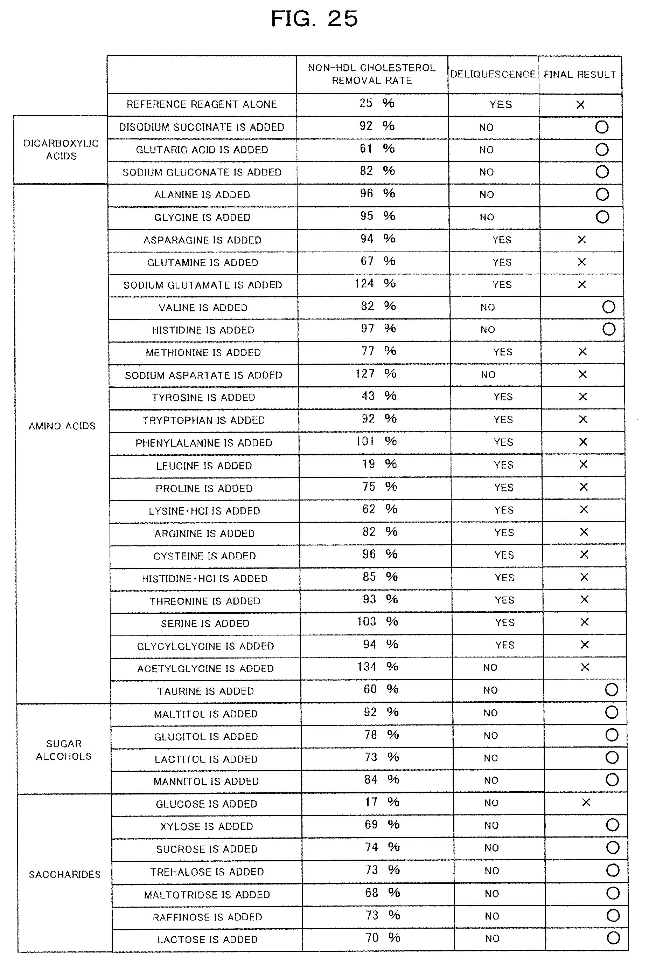

FIGS. 25 to 27 show a second embodiment of the present invention.

A specific example in analysis steps 1 to 7 is identical to that of the first embodiment and thus step 8 and the subsequent steps will be specifically described below. The same constituent elements as in the first embodiment will be indicated by the same reference numerals.

In step 8 after step 7, the rotation of a turntable 101 is stopped, an analysis device 1 is set at the position of FIG. 17(a), and then the turntable 101 is controlled at a frequency of 60 Hz to 120 Hz so as to oscillate the analysis device 1 by about ±1 mm, so that diluted plasma 40 retained in a reserving cavity 53 is transferred to an operation cavity 61 through a connecting section 59 by the action of a capillary force. The connecting section 59 is formed on the side wall of the reserving cavity 53 so as to be immersed under the liquid level of the diluted plasma 40.

Moreover, the turntable 101 is controlled at a frequency of 10 Hz to 40 Hz to agitate the diluted plasma 40 and reagents 67a and 67b carried in the operation cavity 61 illustrated in FIG. 24(a), so that a specific component in the diluted plasma 40 is reacted with the reagents. In this case, the operation cavity 61 is the passage of a microchannel structure before a measuring cell 52a.

The diluted plasma 40 transferred to measuring cells 52b and 52c is, as illustrated in FIG. 17(a), sucked into capillary areas 56b and 56c by a capillary force. At this point, reagents 58b1, 58b2, 58b3, 58c1, and 58c2 start dissolving and the specific component in the diluted plasma 40 starts reacting with the reagents.

The turntable 101 is then rotationally driven in a clockwise direction (direction C2). At this point, as illustrated in FIG. 17(b), the diluted plasma having reacted with the reagents of the operation cavity 61 passes through a connecting passage 64 and flows into a separating cavity 66. Moreover, the high-speed rotation is kept to centrifugally separate aggregates generated in the operation cavity 61. In the present embodiment, when a component to be inspected is reacted with the reagents, a component inhibiting the reaction is removed in an upstream process. The diluted plasma is reacted with the reagents in the operation cavity 61, so that a specific component inhibiting a reaction in a downstream process is coagulated and then the aggregates are removed by centrifugal separation in the subsequent process.

A mixed solution of the reagents retained in the capillary areas 56b and 56c and the diluted plasma is transferred to the outer peripheries of the measuring cells 52b and 52c by a centrifugal force, so that the reagents and the diluted plasma are agitated.

Then, the rotation of the turntable 101 is stopped. At this point, the diluted plasma 40 is sucked into a capillary cavity 69 formed on the wall surface of the separating cavity 66 and flows, as illustrated in FIG. 18(a), into a measuring passage 80 through a connecting passage 70 communicating with the capillary cavity 69, so that a fixed quantity of the diluted plasma is retained.

Moreover, the diluted plasma 40 containing the aggregates in the separating cavity 66 is sucked into a siphon-shaped connecting passage 68 that connects the separating cavity 66 and an overflow cavity 81a.

The mixed solution of the reagents and the diluted plasma in the measuring cells 52b and 52c is sucked into the capillary areas 56b and 56c again by a capillary force.

When the turntable 101 is rotationally driven in the clockwise direction (direction C2), as illustrated in FIG. 18(b), the diluted plasma 40 retained in the measuring passage 80 overflows at the position of a bending section 84 that is connected to an opened-to-atmosphere cavity 83 communicating with the atmosphere, and then only a fixed quantity of the diluted plasma 40 flows into the measuring cell 52a.

The diluted plasma 40 in the separating cavity 66, the connecting passage 70, and the capillary cavity 69 flows into the overflow cavity 81a through the siphon-shaped connecting passage 68.

The mixed solution of the reagents retained in the capillary areas 56b and 56c and the diluted plasma is transferred to the outer peripheries of the measuring cells 52b and 52c by a centrifugal force, so that the reagents and the diluted plasma are agitated.

The rotation of the turntable 101 is then stopped, so that as illustrated in FIG. 19(a), the diluted plasma 40 transferred to the measuring cell 52a is sucked into a capillary area 56a by a capillary force. At this point, reagents 58a1 and 58a2 start dissolving and then the specific component in the diluted plasma 40 starts reacting with the reagents.

Moreover, a mixed solution of the reagents and the diluted plasma in the measuring cells 52b and 52c is sucked into the capillary areas 56b and 56c again by a capillary force.

When the turntable 101 is rotationally driven in the clockwise direction (direction C2), as illustrated in FIG. 19(b), a mixed solution of the reagents retained in the capillary areas 56a, 56b, and 56c and the diluted plasma is transferred to the outer peripheries of the measuring cells 52a, 52b, and 52c by a centrifugal force, so that the reagents and the diluted plasma are agitated.

The analysis device 1 is rotationally driven in a counterclockwise direction (direction C1) or the clockwise direction (direction C2). When the measuring cells 52a, 52b, and 52c pass between a light source 112 and a photodetector 113, an arithmetic unit 110 reads a detected value of the photodetector 113 and calculates the concentration of the specific component.

In the case where a fixed quantity of HDL cholesterol is measured in the measuring cell 52a, the reagents 67a and 67b are prepared as follows:

In order to remove non-HDL, which is lipoprotein other than HDL, from a blood specimen, polyanion and bivalent cation are necessary. In consideration of solubility and the necessity for stable setting on an analysis device in a solid state, at least one compound is selected from the group consisting of phosphotungstic acid, phosphotungstate, phosphomolybdic acid, and molybdophosphate. As a bivalent cation, as has been discussed, at least one substance can be selected from the group consisting of calcium and magnesium, ions. In consideration of a reaction of an enzyme in the analysis device, metal ions that may deactivate the enzyme are not desirable. In view of the difficulty level of availability, calcium or magnesium is selected. Magnesium is desirable for solubility. Sulfate, that is, magnesium sulfate is desirably used for deliquescence. Moreover, calcium sulfate is desirably added to the mixture of phosphotungstate and magnesium sulfate, which changes a crystalline state to improve the solubility of the reagents.

phosphotungstate 20 mg/ml
magnesium sulfate 40mg/ml
calcium sulfate 12 mM
disodium succinate 15 mg/ml
Non-HDL was removed such that a reagent of the composition was prepared, 20 µl of the reagent was dropped into a test tube, and then the reagent was dried. A blood specimen collected from an ordinary person was diluted 1:4 with phosphate buffered saline (pH 7.4), 200 µl of the specimen was added to the dried reagent, the specimen was agitated by a vortex mixer for 45 seconds and was allowed to stand for 75 seconds, and then generated non-HDL aggregates were centrifugally separated at 1500 G for 30 seconds. In the case where the specimen is diluted at least 1:2, the reagents of the present embodiment can be used as they are. In the case where the specimen is diluted 1:2 or less, the reagents can be used by adjusting the component concentrations of the reagents. A supernatant fluid free from non-HDL was collected and cholesterol (corresponding to HDL cholesterol) in a liquid was measured using 7020 automatic analyzer of Hitachi High-Technologies Corporation and "Cholestest-CHO" of SEKISUI MEDICAL CO., LTD..

The deliquescence of the reagent is decided as follows: the reagent carried in a dry state on a resin substrate was exposed at a temperature of 30°C and a humidity of 80% for 30 minutes, and then a centrifugal force of 500 G was applied in the horizontal direction of the resin substrate to decide the presence or absence of deliquescence depending on whether or not the reagent had flown in the direction of the centrifugal force. In the decision of deliquescence, an important decision criterion is the absence of splashes of the reagent under a centrifugal force because the analysis device 1 uses a centrifugal force for, for example, internal transportation of a specimen.

Regarding a removal rate of non-HDL cholesterol and the presence or absence of deliquescence in this process, FIG. 25 shows comparisons among the reagents, a reference reagent not containing disodium succinate, and a reagent containing an additive other than disodium succinate.

Additives shown in FIG. 25 were used in experiments.

For dicarboxylic acids, experiments were conducted on disodium succinate, glutaric acid, and sodium gluconate.

For amino acids, experiments were conducted on alanine, glycine, asparagine, glutamine, sodium glutamate, valine, histidine, methionine, sodium aspartate, tyrosine, tryptophan, phenylalanine, leucine, proline, lysine·HCI, arginine, cysteine, histidine·HCI, threonine, serine, glycylglycine, acetylglycine, and taurine that is an amino acid-like compound.

For sugar alcohols, experiments were conducted on maltitol, glucitol, lactitol, and mannitol.

For saccharides, experiments were conducted on glucose and xylose that are monosaccharides, sucrose and trehalose that are disaccharides, and maltotriose, raffinose, and lactose that are trisaccharides.

In the case where the removal rate of non-HDL cholesterol largely exceeds 100%, it is assumed that an excessive or abnormal reaction occurs so as to remove HDL cholesterol as well. For deliquescence, the type and concentration of the additive are important factors. The column of deliquescence in FIG. 25 indicates, for reference, additive concentration conditions that hardly cause deliquescence. Many effective additives have non-HDL cholesterol removal rates exceeding 25%, which is the removal rate of the reference reagent containing no additives. It is important to remove non-HDL cholesterol in a specimen in a shorter time and eliminate deliquescence for stable setting on the analysis device.

As shown in FIG. 25, the known reference reagent not containing disodium succinate has an insufficient non-HDL cholesterol removal rate of 25% with deliquescence, whereas the reagent containing disodium succinate has a high non-HDL cholesterol removal rate of 92% without causing deliquescence.

The removal rate of non-HDL cholesterol in the present patent is calculated by (total cholesterol concentration - cholesterol concentration after pretreatment)/(total cholesterol concentration - HDL cholesterol concentration true value) x 100. As the removal rate is closer to 100%, the additive is more effective. Since the numerical value depends upon the pretreatment conditions, the numerical value is relatively interpreted. Hence, in the case of application to the analysis device of the present embodiment, an evaluation of correlation to an HDL cholesterol true value proves that the additive having a non-HDL cholesterol removal rate of 100±20% satisfies the standard (a coefficient of determination > 0.975) of CRMLN (Cholesterol Reference Method Laboratory Network), which is an international certification organization of cholesterol. Thus, except for disodium succinate, the optimum additives for the analysis device of the present embodiment are sodium gluconate, alanine, and glycine or valine, histidine, maltitol, and mannitol which can achieve high removal rates and eliminate deliquescence.

Disodium succinate, sodium gluconate, alanine, and glycine or valine and histidine effectively reduce deliquescence at a concentration of at least 5 mg/ml. Maltitol and mannitol effectively reduce deliquescence at a concentration of 1 mg/ml to 10 mg/ml.

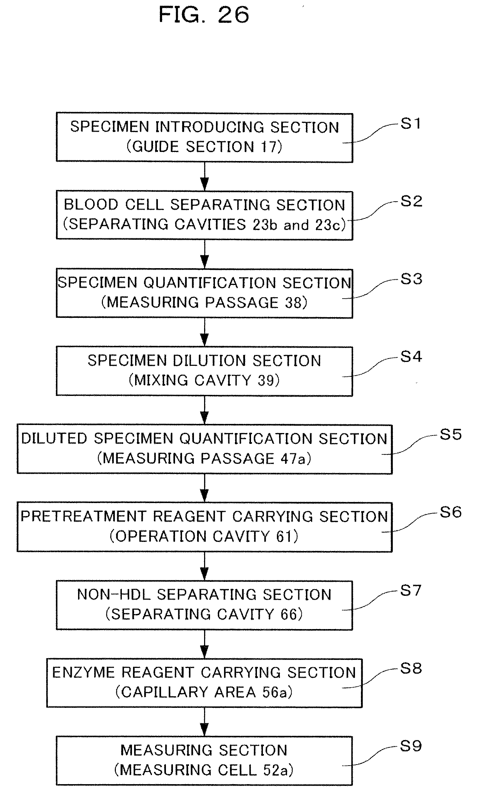

FIG. 26 shows that the reagent is set in a solid state on the operation cavity 61 of the analysis device 1 and then the concentration of HDL cholesterol is measured.

First, in step S1, a blood specimen is introduced into a guide section 17 serving as a specimen introducing section. In the introduction of the blood specimen, blood collected from a fingertip may be directly dropped into an inlet section 13 or blood collected into a blood collection tube by a syringe or the like may be transferred from the blood collection tube into the inlet section 13 by instruments such as a pipet.

In step S2, the introduced blood specimen is transferred to separating cavities 23b and 23c serving as blood cell separating sections, and then blood cell components and plasma components are separated by a centrifugal force. The blood specimen is not limited to whole blood. Blood serum may be introduced instead. In this case, the separating cavities 23b and 23c may be omitted. The specimen is transferred by a combination of a capillary force, a siphon structure, and a centrifugal force.

In step S3, the plasma components separated by the separating cavities 23b and 23c are transferred to a measuring passage 38 serving as a specimen quantification section, and then any quantity of the specimen is quantified and collected.

In step S4, the quantified specimen is transferred to a mixing cavity 39 serving as a specimen dilution section, and then the specimen is diluted to any dilution ratio. The dilution ratio of the specimen is set by, for example, the detection sensitivity of an analysis system and a fluid volume required for the analysis device.

In step S5, the diluted specimen is transferred to a measuring passage 47a serving as a diluted specimen quantification section, and then any quantity of the specimen is quantified and collected from the diluted specimen.

In step S6, the quantified specimen is transferred to the operation cavity 61 serving as a pretreatment reagent carrying section set in a solid state. Non-HDL is coagulated by the reagents 67a and 67b of the operation cavity 61.

In step S7, the coagulated non-HDL is transferred to the separating cavity 66 serving as a non-HDL separating section, and then non-HDL aggregates are removed by a centrifugal force.

In step 8, a supernatant fluid containing HDL is transferred to the capillary area 56a serving as an enzyme reagent carrying section. In the series of separating and removing operations of non-HDL, the specimen is agitated for 60 seconds and then is centrifugally separated at 500 G for 30 seconds without standing. In the capillary area 56a, the reagents 58a1 and 58a2 including a known enzyme and chromogen are set in a solid state. The reagents 58a1 and 58a2 specifically react with cholesterol and develop colors according to the concentration of cholesterol.

In step S9, the specimen having developed a color according to the concentration of HDL cholesterol in the capillary area 56a is transferred to the measuring cell 52a serving as a measuring section, and then the degree of coloring is determined by measuring the absorbance of light emitted from a measuring apparatus. The absorbance of the specimen is converted to a cholesterol concentration according to a prepared calibration curve, so that the concentration of HDL cholesterol in the specimen can be determined.

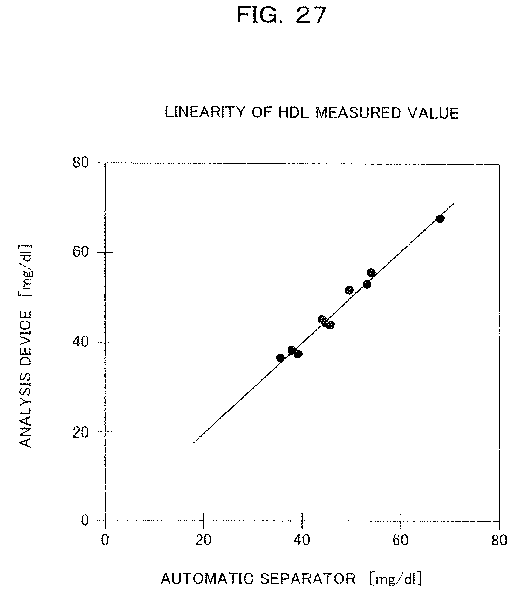

FIG. 27 shows the measurement results of HDL cholesterol concentrations of a blood specimen collected from an ordinary person. The concentrations were measured using the analysis device 1. FIG. 27 shows a reference value that is a value measured by 7020 automatic analyzer of Hitachi High-Technologies Corporation and "Cholestest N-HDL" that is an HDL-cholesterol kit of SEKISUI MEDICAL CO., LTD..

HDL cholesterol concentrations measured by the analysis device 1 using the reagent have excellent linearity with a correlation coefficient of 0.979 relative to a reference value, achieving sufficient measurement capability of HDL cholesterol concentrations even in the case of short-time pretreatment. Moreover, the reagent is set in a solid state on the analysis device, thereby reducing the size of the analysis device. In the present embodiment, an HDL cholesterol concentration can be automatically measured with a small quantity of specimen, not more than ten microliters, with high accuracy in a short time, thereby effectively improving the quality of medical treatment and reducing the burden of a patient as defined in POCT.

The optimum additive is selected on the condition that the non-HDL cholesterol removal rate is in the range of 100±20%. In view of the reference reagent having a non-HDL cholesterol removal rate of 25%, even when a requirement for selection is relaxed to a non-HDL cholesterol removal rate of 100+20% exceeding 25%, an analyzing apparatus can be obtained with improved performance. The relaxation of the requirement allows the selection of glutaric acid, taurine, glucitol, lactitol, xylose, sucrose, trehalose, maltotriose, raffinose, and lactose as additives in the experimental results of FIG. 25. The additives additionally selected in the relaxation of the requirement have concentrations of at least 5 mg/ml or 5 mg/ml to 20 mg/ml. Specifically, glutaric acid, taurine, lactitol, xylose, sucrose, trehalose, maltotriose, raffinose, and lactose have concentrations of at least 5 mg/ml and glucitol has a concentration of about 5mg/ml to 20mg/ml.

In the experimental example of FIG. 25, any ones of the additives were added to prepare the reagents 67a and 67b serving as pretreatment reagents. The reagents 67a and 67b may contain any ones of the effective additives or at least one of the compounds of the additives.

In consideration of the conditions of the performed steps, proper analysis reagents are selected from alternatives under the following selecting conditions: a reagent containing a polyanionic compound, a bivalent cationic compound, and at least one compound is contacted with a biological sample in a dry state, is agitated for 45 seconds, and then is allowed to stand for 75 seconds, the removal rate of non-high-density lipoprotein cholesterol in a supernatant fluid is 100±20% after generated non-HDL aggregates are centrifugally separated at 1500 G for 30 seconds, and deliquescence is not recognized after centrifugal separation at 500 G for five minutes at 30 degrees Celsius and a humidity of 80%.

### Industrial Applicability

The present invention can contribute to size reduction and improved performance of an analysis device used for analyzing a component of a liquid collected from an organism or the like.

## Claims

1. An analysis reagent that coagulates lipoprotein other than high-density lipoprotein in an analysis of high-density lipoprotein cholesterol contained in a biological sample;
wherein the reagent comprising a combination of a polyanionic compound and a bivalent cationic compound contains one substance selected from the group consisting of succinic acid, gluconic acid, alanine, glycine, valine, histidine, maltitol and mannitol or a salt thereof;
wherein the polyanionic compound is at least one selected from the group consisting of phosphotungstic acid, phosphotungstate, phosphomolybdic acid, and molybdophosphate; and
wherein the bivalent cationic compound is at least one selected from the group consisting of a magnesium ion compound, magnesium ion compound salt, a calcium ion compound, and calcium ion compound salt.

2. An analysis device having a microchannel structure for transferring a sample liquid to a measuring cell by a centrifugal force, the analysis device being used for reading that accesses a reaction liquid in the measuring cell,
wherein an analysis reagent according to claim 1 in a solid state is carried in a passage of the microchannel structure before reaching the measuring cell.

3. The analysis device according to claim 2, comprising:
a reserving cavity that receives a fixed quantity of diluted plasma serving as a liquid sample;
an operation cavity that is formed next to the reserving cavity in a circumferential direction, is connected to the reserving cavity via a connecting section enabling application of a capillary force, and contains a reagent for analyzing high-density lipoprotein cholesterol;
a separating cavity that is formed outside the operation cavity and is connected to the operation cavity via a connecting passage serving as a clearance enabling application of a capillary force;
a measuring passage that is adjacent to the separating cavity in the circumferential direction and is connected to the separating cavity via a connecting passage enabling application of a capillary force;
a measuring cell formed outside the measuring passage; and
a capillary area that is formed inside the measuring cell and contains an enzyme reagent and a mediator,
wherein a reaction solution having reacted in the capillary area is transferred to an outer periphery of the measuring cell by increasing the centrifugal force, and then the reaction solution retained on the outer periphery of the measuring cell is accessed to measure HDL of the liquid sample.

4. The analysis device according to claim 3, wherein the operation cavity comprises a reagent carrying section that carries the reagent for analyzing high-density lipoprotein cholesterol, the reagent carrying section protruding from the operation cavity so as to have a clearance smaller than a clearance of the operation cavity.

5. The analysis device according to claim 4, further comprising an agitating rib extended in a radial direction in the operation cavity, the agitating rib being lower in height than an external wall of the operation cavity.

## Patentansprüche

1. Analysereagenz, das anderes Lipoprotein als HDL (High Density Lipoprotein) bei einer
Analyse von HDL-Cholesterin in einer biologischen Probe koaguliert,
wobei das Reagenz, das eine Kombination aus einer polyanionischen Verbindung und einer bivalenten kationischen Verbindung ist, eine Substanz enthält, die aus der Gruppe ausgewählt ist, die Bernsteinsäure, Gluconsäure, Alanin, Glycin, Valin, Histidin, Maltit und Mannit oder ein Salz derselben umfasst,
wobei die polyanionische Verbindung wenigstens eine ist, die aus der Gruppe ausgewählt ist, die Wolframotophosphorsäure, Phosphorwolframat, Molybdatophosphorsäure und Molybdophosphat umfasst, und
wobei die bivalente kationische Verbindung wenigstens eine ist, die aus der Gruppe ausgewählt ist, die eine Magnesiumionenverbindung, ein Magnesiumionenverbindungssalz, eine Calciumionenverbindung und ein Calciumionenverbindungssalz umfasst.

2. Analysevorrichtung, die einen Mikrokanalaufbau für das Transportieren einer Probenflüssigkeit zu einer Messzelle durch eine Zentrifugalkraft aufweist, wobei die Analysevorrichtung für eine Messung verwendet wird, die auf eine Reaktionsflüssigkeit in der Messzelle zugreift,
wobei ein Analysereagenz nach Anspruch 1 in einem festen Zustand in einem Durchgang des Mikrokanalaufbaus getragen wird, bevor es die Messzelle erreicht.

3. Analysevorrichtung nach Anspruch 2, die umfasst:
einen Aufnahmehohlraum der eine fixe Menge eines verdünnten Plasmas aufnimmt, das als eine flüssige Probe dient,
einen Operationshohlraum, der neben dem Aufnahmehohlraum in einer Umfangsrichtung ausgebildet ist, mit dem Aufnahmehohlraum über einen Verbindungsabschnitt verbunden ist, um die Ausübung einer Kapillarkraft zu gestatten, und ein Reagenz für das Analysieren von HDL-Cholesterin enthält,
einen Trennhohlraum, der außerhalb des Operationshohlraums ausgebildet ist und mit dem Operationshohlraum über einen Verbindungsdurchgang verbunden ist, der als ein Freiraum dient, um die Ausübung einer Kapillarkraft zu gestatten,
einen Messdurchgang, der dem Trennhohlraum in der Umfangsrichtung benachbart ist und mit dem Trennhohlraum über einen Verbindungsdurchgang verbunden ist, um eine Ausübung einer Kapillarkraft zu gestatten,
eine Messzelle, die außerhalb des Messdurchgangs ausgebildet ist, und
einen Kapillarbereich, der innerhalb der Messzelle ausgebildet ist und ein Enzymreagenz und einen Mediator enthält,
wobei eine Reaktionslösung, die in dem Kapillarbereich reagiert hat, zu einem Außenumfang der Messzelle transportiert wird, indem die Zentrifugalkraft erhöht wird, und wobei dann auf die an dem Außenumfang der Messzelle gehaltene Reaktionslösung zugegriffen wird, um das HDL der flüssigen Probe zu messen.

4. Analysevorrichtung nach Anspruch 3, wobei der Operationshohlraum einen Reagenztrageabschnitt umfasst, der das Reagenz für das Analysieren von HDL-Cholesterin trägt, wobei der Reagenztrageabschnitt von dem Operationshohlraum vorsteht, sodass er einen kleineren Freiraum aufweist als der Operationshohlraum.

5. Analysevorrichtung nach Anspruch 4, die weiterhin eine Erregungsrippe umfasst, die sich in einer Radialrichtung in dem Operationshohlraum erstreckt, wobei die Erregungsrippe eine kleinere Höhe aufweist als eine Außenwand des Operationshohlraums.

## Revendications

1. Réactif d'analyse qui coagule les lipoprotéines autres que les lipoprotéines de haute densité dans une analyse du cholestérol des lipoprotéines de haute densité contenu dans un échantillon biologique ;
dans lequel le réactif comprenant une association d'un composé polyanionique et d'un composé cationique bivalent contient une substance sélectionnée dans le groupe constitué par l'acide succinique, l'acide gluconique, l'alanine, la glycine, la valine, le maltitol et le mannitol ou un sel de ceux-ci ;
dans lequel le composé polyanionique est au moins un composé sélectionné dans le groupe constitué par l'acide phosphotungstique, le phosphotungstate, l'acide phosphomolybdique, et le molybdophosphate ; et
dans lequel le composé cationique bivalent est au moins un composé sélectionné dans le groupe constitué par un composé d'ion magnésium, un sel de composé d'ion magnésium, un composé d'ion calcium, et un sel de composé d'ion calcium.

2. Dispositif d'analyse ayant une structure à microcanaux pour le transfert d'un liquide d'échantillon dans une cellule de mesure par une force centrifuge, le dispositif d'analyse étant utilisé pour la lecture de ces entrées de liquide réactionnel dans la cellule de mesure,
dans lequel un réactif d'analyse selon la revendication 1 à l'état solide est transporté dans un conduit de la structure à microcanaux avant d'atteindre la cellule de mesure.

3. Dispositif d'analyse selon la revendication 2, comprenant :
une cavité de réserve qui reçoit une quantité fixe de plasma dilué servant d'échantillon liquide ;
une cavité d'exploitation qui est formée à côté de la cavité de réserve dans une direction circonférentielle, est reliée à la cavité de réserve par l'intermédiaire d'une section de liaison permettant l'application d'une force capillaire, et contient un réactif pour l'analyse du cholestérol des lipoprotéines de haute densité ;
une cavité de séparation qui est formée en dehors de la cavité d'exploitation et est reliée à la cavité d'exploitation par l'intermédiaire d'un conduit de liaison servant d'espace libre permettant l'application d'une force capillaire ;
un conduit de mesure qui est adjacent à la cavité de séparation dans la direction circonférentielle et est relié à la cavité de séparation par l'intermédiaire d'un conduit de liaison permettant l'application d'une force capillaire ;
une cellule de mesure formée en dehors du conduit de mesure ; et
une zone capillaire qui est formée à l'intérieur de la cellule de mesure et contient un réactif enzymatique et un médiateur,
dans lequel une solution réactionnelle ayant réagi dans la zone capillaire est transférée vers une périphérie extérieure de la cellule de mesure par augmentation de la force centrifuge, puis la solution réactionnelle retenue sur la périphérie extérieure de la cellule de mesure fait l'objet d'un accès pour mesurer les lipoprotéines de haute densité (HDL) de l'échantillon liquide.

4. Dispositif d'analyse selon la revendication 3, dans lequel la cavité d'exploitation comprend une section de transport de réactif qui transporte le réactif pour l'analyse du cholestérol des lipoprotéines de haute densité, la section de transport de réactif dépassant de la cavité d'exploitation de façon à avoir un espace libre plus petit qu'un espace libre de la cavité d'exploitation.

5. Dispositif d'analyse selon la revendication 4, comprenant en outre une nervure d'agitation s'étendant dans une direction radiale dans la cavité d'exploitation, la nervure d'agitation ayant une hauteur plus basse qu'une paroi extérieure de la cavité d'exploitation.
